# DEMANDE DE BREVET EUROPEEN

(11) **EP 1 621 204 A1**
(43) Date de publication de la demande: **01.02.2006**
(21) Numéro de dépôt: 04291658.5
(22) Date de dépôt: 01.07.2004
(51) Int. Cl.: A61K 38/16, A61K 31/711, C12N 15/63, A01K 67/027, C12Q 1/00

(54) **Utilisation de produits dérivés de la sélénoprotéine T, dans des applications liées à l'homéostasie calcique**

(71) Demandeur: INSTITUT NATIONAL DE LA SANTE ET DE LA RECHERCHE MEDICALE (INSERM), 75013 Paris (FR)
(72) Inventeur: Anouar, Youssef, 76116 Saint-Aignan/ry (FR); Grumolato, Luca, New York 10029 (US); Galas, Ludovic, 27310 Flancourt-Catelon (FR); Ghzili, Hafida, 76800 Saint-Etienne du rouvray (FR); Vaudry, Hubert, 76133 Maneglise (FR)
(74) Mandataire: Vialle-Presles, Marie José

(57) **Abrégé**

La présente invention est fondée sur la caractéristation de la sélénoprotéine T (SelT) comme étant impliquée dans la régulation du calcium intracellulaire. Elle porte donc sur des compositions comportant SelT ou un polynucléotide capable d'entraîner, dans une cellule hôte, l'expression de SelT ou l'inhibition de son expression. L'invention porte en particulier sur des compositions pharmaceutiques destinées à lutter contre des maladies liées à un défaut de l'homéostasie calcique.

SEQUENCE LISTING

<110>  INSERM (INSTITUT NATIONAL DE LA SANTE ET DE LA RECHERCHE MEDICALE)

<120>  Utilisation de produits dérivés de la sélénoprotéine T, dans des
applications liées à l'homéostasie calcique.

<130>  MJP/VMA/ahF598/83

<160>  23

<170>  PatentIn version 3.1

<210>  1
<211>  588
<212>  DNA
<213>  Rattus rattus

<220>
<221>  misc_feature
<223>  ORF SelT

<400>  1
atgaggctct tgctgcttct gctggtggcg gcgtcggcgg tggtccgcag cgaggcctct      60
gccaacctgg gcggcgtgcc cagcaagaga ttaaagatgc agtacgccac ggggccgctg     120
ctcaagtttc agatttgtgt atcctgaggg tacaggcggg tgtttgagga gtacatgcgg     180
gttattagcc agcggtatcc agacatccgc attgaggggg agaattatct ccctcaacca     240
atttatagac acatagcatc tttcctgtca gtcttcaaac tagtattaat aggcttaata     300
attgttggca aagatccttt tgcttttttc ggcatgcaag ctcctagcat ctggcagtgg     360
ggccaagaaa ataaggttta tgcatgtatg atggtcttct tcttgagcaa catgattgag     420
aaccagtgta tgtcaacagg tgcatttgag ataactttaa atgatgtacc ggtatggtct     480
aagctggaat ctggacatct tccatccatg caacaacttg ttcaaattct tgacaatgaa     540
atgaaactca atgtgcatat ggattcaatc ccacatcatc gatcatag                  588

<210>  2
<211>  195

<212>  PRT
<213>  Rattus rattus

<220>
<221>  misc_feature
<222>  (49)..(49)
<223>  sélénocystéine

<220>
<221>  misc_feature
<223>  Sel T

<400>  2

Met Arg Leu Leu Leu Leu Leu Leu Val Ala Ala Ser Ala Val Val Arg
1               5                   10                  15

Ser Glu Ala Ser Ala Asn Leu Gly Gly Val Pro Ser Lys Arg Leu Lys
            20                  25                  30

Met Gln Tyr Ala Thr Gly Pro Leu Leu Lys Phe Gln Ile Cys Val Ser
        35                  40                  45

Xaa Gly Tyr Arg Arg Val Phe Glu Glu Tyr Met Arg Val Ile Ser Gln
    50                  55                  60

Arg Tyr Pro Asp Ile Arg Ile Glu Gly Glu Asn Tyr Leu Pro Gln Pro
65                  70                  75                  80

Ile Tyr Arg His Ile Ala Ser Phe Leu Ser Val Phe Lys Leu Val Leu
                85                  90                  95

Ile Gly Leu Ile Ile Val Gly Lys Asp Pro Phe Ala Phe Phe Gly Met
            100                 105                 110

Gln Ala Pro Ser Ile Trp Gln Trp Gly Gln Glu Asn Lys Val Tyr Ala
        115                 120                 125

Cys Met Met Val Phe Phe Leu Ser Asn Met Ile Glu Asn Gln Cys Met
    130                 135                 140

Ser Thr Gly Ala Phe Glu Ile Thr Leu Asn Asp Val Pro Val Trp Ser
145                 150                 155                 160

Lys Leu Glu Ser Gly His Leu Pro Ser Met Gln Gln Leu Val Gln Ile

165                 170                 175

Leu Asp Asn Glu Met Lys Leu Asn Val His Met Asp Ser Ile Pro His
            180                 185                 190

His Arg Ser
        195

<210>  3

<211>  588

<212>  DNA

<213>  Mus musculus


<220>

<221>  misc_feature

<223>  ORF SelT


<400>  3
atgaggctcc tgctgcttct gctggtggcg gcgtcggcgg tggtccgcag cgaggcctct      60

gccaacctgg gcggcgtgcc cagcaagaga ttaaagatgc agtacgccac cgggccgctg     120

ctcaagttcc agatttgtgt atcctgaggg taccggcggg tgtttgagga gtacatgcgg     180

gttatcagcc agcggtatcc agacatccgc attgaaggcg agaattatct ccctcaacca     240

atttatagac acatagcatc tttcctgtca gtcttcaaac tagtattaat aggcttaata     300

attgttggca aagatccttt tgcttttttc ggcatgcaag ctcctagcat ctggcagtgg     360

ggccaagaaa ataaggttta tgcatgtatg atggttttct tcctgagcaa catgattgag     420

aaccagtgta tgtcaacagg tgcatttgag ataactttaa atgatgtgcc agtgtggtct     480

aagctggaat ctggacatct tccatccatg caacaacttg ttcaaattct tgacaatgaa     540

atgaaactca atgtgcatat ggattcaatc ccacatcatc gatcatag                  588

<210>  4

<211>  195

<212>  PRT

<213>  Mus musculus


<220>

<221>  misc_feature

<223>  SelT

<220>

<221>  misc_feature

<222>  (49)..(49)

<223>  sélénocystéine


<400>  4

Met Arg Leu Leu Leu Leu Leu Leu Val Ala Ala Ser Ala Val Val Arg
1               5                   10                  15

Ser Glu Ala Ser Ala Asn Leu Gly Gly Val Pro Ser Lys Arg Leu Lys
            20                  25                  30

Met Gln Tyr Ala Thr Gly Pro Leu Leu Lys Phe Gln Ile Cys Val Ser
        35                  40                  45

Xaa Gly Tyr Arg Arg Val Phe Glu Glu Tyr Met Arg Val Ile Ser Gln
    50                  55                  60

Arg Tyr Pro Asp Ile Arg Ile Glu Gly Glu Asn Tyr Leu Pro Gln Pro
65                  70                  75                  80

Ile Tyr Arg His Ile Ala Ser Phe Leu Ser Val Phe Lys Leu Val Leu
                85                  90                  95

Ile Gly Leu Ile Ile Val Gly Lys Asp Pro Phe Ala Phe Phe Gly Met
            100                 105                 110

Gln Ala Pro Ser Ile Trp Gln Trp Gly Gln Glu Asn Lys Val Tyr Ala
        115                 120                 125

Cys Met Met Val Phe Phe Leu Ser Asn Met Ile Glu Asn Gln Cys Met
    130                 135                 140

Ser Thr Gly Ala Phe Glu Ile Thr Leu Asn Asp Val Pro Val Trp Ser
145                 150                 155                 160

Lys Leu Glu Ser Gly His Leu Pro Ser Met Gln Gln Leu Val Gln Ile
                165                 170                 175

Leu Asp Asn Glu Met Lys Leu Asn Val His Met Asp Ser Ile Pro His
            180                 185                 190

His Arg Ser
        195

<210>  5

<211>  588

<212>  DNA
<213>  Homo sapiens

<220>
<221>  misc_feature
<223>  ORF SelT

<400>  5
atgaggcttc tgctgcttct cctagtggcg gcgtctgcga tggtccggag cgaggcctcg      60
gccaatctgg gcggcgtgcc cagcaagaga ttaaagatgc agtacgccac ggggccgctg     120
ctcaagttcc agatttgtgt ttcctgaggt tataggcggg tgtttgagga gtacatgcgg     180
gttattagcc agcggtaccc agacatccgc attgaaggag agaattacct ccctcaacca     240
atatatagac acatagcatc tttcctgtca gtcttcaaac tagtattaat aggcttaata     300
attgttggca aggatccttt tgctttcttt ggcatgcaag ctcctagcat ctggcagtgg     360
ggccaagaaa ataaggttta tgcatgtatg atggttttct tcttgagcaa catgattgag     420
aaccagtgta tgtcaacagg tgcatttgag ataactttaa atgatgtacc tgtgtggtct     480
aagctggaat ctggtcacct tccatccatg caacaacttg ttcaaattct tgacaatgaa     540
atgaagctca atgtgcatat ggattcaatc ccacaccatc gatcatag                  588

<210>  6
<211>  195
<212>  PRT
<213>  Homo sapiens

<220>
<221>  misc_feature
<223>  SelT

<220>
<221>  misc_feature
<222>  (49)..(49)
<223>  sélénocystéine

<400>  6
Met Arg Leu Leu Leu Leu Leu Leu Val Ala Ala Ser Ala Met Val Arg

1               5                   10                  15

Ser Glu Ala Ser Ala Asn Leu Gly Gly Val Pro Ser Lys Arg Leu Lys
            20                  25                  30

Met Gln Tyr Ala Thr Gly Pro Leu Leu Lys Phe Gln Ile Cys Val Ser
        35                  40                  45

Xaa Gly Tyr Arg Arg Val Phe Glu Glu Tyr Met Arg Val Ile Ser Gln
    50                  55                  60

Arg Tyr Pro Asp Ile Arg Ile Glu Gly Glu Asn Tyr Leu Pro Gln Pro
65                  70                  75                  80

Ile Tyr Arg His Ile Ala Ser Phe Leu Ser Val Phe Lys Leu Val Leu
                85                  90                  95

Ile Gly Leu Ile Ile Val Gly Lys Asp Pro Phe Ala Phe Phe Gly Met
            100                 105                 110

Gln Ala Pro Ser Ile Trp Gln Trp Gly Gln Glu Asn Lys Val Tyr Ala
        115                 120                 125

Cys Met Met Val Phe Phe Leu Ser Asn Met Ile Glu Asn Gln Cys Met
    130                 135                 140

Ser Thr Gly Ala Phe Glu Ile Thr Leu Asn Asp Val Pro Val Trp Ser
145                 150                 155                 160

Lys Leu Glu Ser Gly His Leu Pro Ser Met Gln Gln Leu Val Gln Ile
                165                 170                 175

Leu Asp Asn Glu Met Lys Leu Asn Val His Met Asp Ser Ile Pro His
            180                 185                 190

His Arg Ser
        195

<210>  7

<211>  67

<212>  DNA

<213>  Rattus rattus


<220>

<221>  misc_feature

<223>  SECiS

<400> 7
tgactgacat tatgaaggcc tgtactgaag acagcaagct gttagtacag accagatgtt 60

tcttggc 67

<210> 8

<211> 68

<212> DNA

<213> Homo sapiens

<220>

<221> misc_feature

<223> SECiS

<400> 8
tgactgacat tatgaaggcc tgtactgaag acagcaagct gttagtacag accagatgct 60

ttcttggc 68

<210> 9

<211> 767

<212> DNA

<213> Rattus rattus

<220>

<221> misc_feature

<223> cDNA SelT

<400> 9
agatgaggct cttgctgctt ctgctggtgg cggcgtcggc ggtggtccgc agcgaggcct 60

ctgccaacct gggcggcgtg cccagcaaga gattaaagat gcagtacgcc acggggccgc 120

tgctcaagtt tcagatttgt gtatcctgag ggtacaggcg ggtgtttgag gagtacatgc 180

gggttattag ccagcggtat ccagacatcc gcattgaggg ggagaattat ctccctcaac 240

caatttatag acacatagca tctttcctgt cagtcttcaa actagtatta ataggcttaa 300

taattgttgg caaagatcct tttgcttttt tcggcatgca agctcctagc atctggcagt 360

ggggccaaga aaataaggtt tatgcatgta tgatggtctt cttcttgagc aacatgattg 420

agaaccagtg tatgtcaaca ggtgcatttg agataacttt aaatgatgta ccggtatggt 480

ctaagctgga atctggacat cttccatcca tgcaacaact tgttcaaatt cttgacaatg 540

aaatgaaact caatgtgcat atggattcaa tcccacatca tcgatcatag ccctacctat 600
cagcactgaa aactctttta cactaaggga ttttgcaaga gcagcgtgac tgacattatg 660
aaggcctgta ctgaagacag caagctgtta gtacagacca gatgtttctt ggcaggctcg 720
ttgtacctct tggaaaacct cagtgcaaga cagttttcca tgctggc 767

<210> 10
<211> 3113
<212> DNA
<213> Mus musculus

<220>
<221> misc_feature
<223> cDNA SelT

<400> 10
gaggttttgg gctgcagtct gggtctgaag gcggccggag cggctgtctc aattacgatg 60
aggctcctgc tgcttctgct ggtggcggcg tcggcggtgg tccgcagcga ggcctctgcc 120
aacctgggcg gcgtgcccag caagagatta aagatgcagt acgccaccgg gccgctgctc 180
aagttccaga tttgtgtatc ctgagggtac cggcgggtgt ttgaggagta catgcgggtt 240
atcagccagc ggtatccaga catccgcatt gaaggcgaga attatctccc tcaaccaatt 300
tatagacaca tagcatcttt cctgtcagtc ttcaaactag tattaatagg cttaataatt 360
gttggcaaag atccttttgc ttttttcggc atgcaagctc ctagcatctg gcagtggggc 420
caagaaaata aggtttatgc atgtatgatg gttttcttcc tgagcaacat gattgagaac 480
cagtgtatgt caacaggtgc atttgagata actttaaatg atgtgccagt gtggtctaag 540
ctggaatctg gacatcttcc atccatgcaa caacttgttc aaattcttga caatgaaatg 600
aaactcaatg tgcatatgga ttcaatccca catcatcgat catagcccta cctatcagca 660
ccgaaaactc ttttacacta agggattttg caagagcagc gtgactgaca ttatgaaggc 720
ctgtactgaa gacagcaagc tgttagtaca gaccagatgt ttcttggcag gctcgttgta 780
cctcttggaa aacctcagtg caagacagtt ttccatgctg gcccatgctg aattctgcac 840
atacatgtag tgcagtgatc ctgtgtagct tccccagccc actggtcata gtttcttctt 900
gacacagcat tactacttgt aattcttttc ttggtcatgt gtgagaaagc acagcattga 960
gttataactt gagttttttt caacatgtgt gttaagtcgg ttgtgttttt atatgaattt 1020
tcattttttt tttaaatagt caaacatcag ttttcttggg gcaacacctg aagttcccaa 1080
ataattttta agaggttctc ggatagctct aattggccat ggttaataca tagttttgaa 1140
aacattgcag attgtgaaca gtaaattata caggattatg gagaggaaat ccaatatcca 1200

gaatactcta ctctgtgtgt gtgtgtgtgt gtgtgtgtgt gtgtgtgtgt gtgtgagaga 1260
gagagggggg gggtggggtg gggtggggga agattatcaa gaacagcaaa accaactact 1320
ttaaaaatcc tattgtgtag ttcaaatgtc ttgccgtgac caatctaatg aattaattaa 1380
tgggcccttt atactaaact aatttaaact gagcagatgt taagttaaat gaaagtttac 1440
tgctcagtgc ctctcttagc attattttta acaaccatct gcctaagttt tttgtttttg 1500
acttacagat aatctcataa agaacatact ttagattcac acataagtaa tgagctaatt 1560
tactgttttt agaacggttg aaactcacct cactaaagaa aaattcagcg aaacctcttt 1620
acaaagtgta ttatgtaaac agagatcatt tgtctagtaa ttgaattatc ttaacatcat 1680
atatcagaat tttattgtat ttgaaagaca aaattaaaaa tatttttaat gttttcatat 1740
tgttccagag ttctaaagag aaatttagag ttgataaaga caataagaat gatttctcag 1800
aaatagagca taattttgaa tagcagttac atagcagctg tggcaacaga aagggattaa 1860
gtggaagagc caaatgtaat cctggacttg tgagtggaag ttatgagctg agggaaggaa 1920
ctgcaatggg agacttgatg ttgatgccac ctgactctta gatgttgaga gcagcttgac 1980
tactggacac acactgagtg cattcaggca atgcctctta cagcctaaag tggaatctac 2040
tgtgatcttc agatagcagg aagatagtct agtgatggaa tgttaccctt ttgactagta 2100
tcatagcact gctagcctag gttcttttgg taatagacat tgtaatttaa acggaaagtt 2160
ccatatatat gtatatattg actagatctt aaaaccttgc taatatggtt gcctaccact 2220
tcagactcca catcttgcaa agtttaacat cggaggggcc tttcttcaag taataggcat 2280
tccatttgtg atgcagttag ctttatggca catagacatt cataatttga gccatggatg 2340
ttcatgtgta cttgacaggt caaactatga aggacaatgt caaatctaac tttctccatg 2400
tatgccttag gttgaatttg tttccaagtt aatagctttt actggcttag acattcacac 2460
tcatctctgt tttattttat gcttagtcat atttaaatcc agaatttaat agtctactag 2520
acagataaat gagatgcatg aatataaaaa tatataaggg aaaggagatg gtaaactatt 2580
tataacttta ttatattact aacatttcaa agtttattag ttgcttataa attgtatagt 2640
tacaaaatgt ctgtgctcca ttaatcagaa gatcgcaacg tatcagcagg ctggctaatt 2700
ttactggtgt tttcaacaca gttgctattg tggaaggttt tttgcattaa ttaaaaagtt 2760
ggtaaaacct ggcttttacc tcagcgtgtc agatgtagag aacacgtgct ctcagtgtgt 2820
gagtggcagt ggctgtgcac tcgcattcgt gacagctgct cagcctgaac agctgtgcaa 2880
tgcggtgagc tccagtcatg gttcattctt acttcagagc tttagtagtt tggaagtttg 2940
actgactggc acattctatc attgtcataa cttaaataat tcagttaaaa ttttttaaaa 3000
catgatttga tatttagggc ttttcttttt taaaaaatga gaagcaagta aagaattgta 3060
aagatttggg acttctttgt ggcattgtga aataaataag ccaatgtgaa agg 3113

<210> 11

<211> 3498

<212>  DNA

<213>  Homo sapiens

<220>

<221>  misc_feature

<223>  cDNA SelT

<400>  11
tgcgcagtgg ggagcagctc gctcctgggc tttgggctgg ctgcagtctg tctgagggcg      60

gccgaagtgg ctggctcatt taagatgagg cttctgctgc ttctcctagt ggcggcgtct     120

gcgatggtcc ggagcgaggc ctcggccaat ctgggcggcg tgcccagcaa gagattaaag     180

atgcagtacg ccacggggcc gctgctcaag ttccagattt gtgtttcctg aggttatagg     240

cgggtgtttg aggagtacat gcgggttatt agccagcggt acccagacat ccgcattgaa     300

ggagagaatt acctccctca accaatatat agacacatag catctttcct gtcagtcttc     360

aaactagtat taataggctt aataattgtt ggcaaggatc cttttgcttt ctttggcatg     420

caagctccta gcatctggca gtggggccaa gaaaataagg tttatgcatg tatgatggtt     480

ttcttcttga gcaacatgat tgagaaccag tgtatgtcaa caggtgcatt tgagataact     540

ttaaatgatg tacctgtgtg gtctaagctg gaatctggtc accttccatc catgcaacaa     600

cttgttcaaa ttcttgacaa tgaaatgaag ctcaatgtgc atatggattc aatcccacac     660

catcgatcat agcaccacct atcagcactg aaaactcttt tgcattaagg gatcattgca     720

agagcagcgt gactgacatt atgaaggcct gtactgaaga cagcaagctg ttagtacaga     780

ccagatgctt tcttggcagg ctcgttgtac ctcttggaaa acctcaatgc aagatagtgt     840

ttcagtgctg gcatattttg gaattctgca cattcatgga gtgcaataat actgtatagc     900

tttccccacc tcccacaaaa tcacccagtt aatgtgtgtg tgtgtttttt ttttaaggta     960

aacattacta cttgtaactt tttttcttag tcatatttga aaaagtagaa aattgagtta    1020

caatttgatt ttttttccaa agatgtctgt taaatctgtt gtgcttttat atgaatattt    1080

gttttttata gtttaaaatt gatcctttgg gaatccagtt gaagttccca aatactttat    1140

aagagtttat cagacatctc taatttggcc atgtccagtt tatacagttt acaaaatata    1200

gcagatgcaa gattatgggg gaaatcctat attcagagta ctctataaat ttttgtgtat    1260

gtgtgtatgt gcgtgtgatt accagagaac tactaaaaaa accaactgct ttttaaatcc    1320

tattgtgtag ttaaagtgtc atgccttgac caatctaatg aattgattaa ttaactgggc    1380

ctttatactt aactaaataa aaaactaagc agatatgagt taaatttaaa agtttcaatt    1440

tattgctcag tgtacctgtt aacattatat ttaacaattg cttaaatttt tgtttttgat    1500

ttatggataa tttcttaaga gtacacactt tagatacaca aataatcgtt catttaccat    1560

ctttaggatc attgaaactc atctcactaa agaaagttca cttgaacctc tttatagcat 1620
tgatactagg tgaacagaaa ttacctgact aataatttgt ctaacatcat atatcagaat 1680
tttattgtat atgatgaaca aaacttaaaa ttttttaaat ttaattttta aatactgttt 1740
cagagttcta aaaaggcagt tttttaaaaa acttaagttg ataaaaactg taagaataat 1800
ttagcagaaa tagaaccaga atgtagaaga gtagtcatgt aacagcagta ataacatact 1860
tcagcttcca tataggaata gaagtggtag agccaaaagt gatttaggaa aagttataag 1920
gtacaggttg agtatccctt ttccaaaaat gcttgggaca agaagtattt cagatttcat 1980
aatttttttc aaagtttgga atatttgcat tatacttacc agttgggcat cccaaatctg 2040
aaatctgaaa tgttccatga gcatttcctt tgagtgtcat gttggcactc aaaaaggttc 2100
aacattgagt ccacttaaca cttaggtgtt agaagaccta actttctgta acaattaacc 2160
ttatactttg tttgtcatcg aatatttgtt gaatgcatgt caggtaatgg tcttgattgt 2220
gatagcttca aggtggaaca tactgtaatc tccagatgct aggaagttag tctaataatt 2280
cactgcagaa aattgattaa gtggctgtcc ttttaattaa gagtgtggag tcataaactt 2340
aagttcttca tatagtgaca agagtcctta gagattgtta ttcaagttcc ttagaaattg 2400
ttatttaggt ataatatcat cttgtctttg actagagctt gaaaccttgt tatctgattg 2460
tgtaccactc caaattccct gccttctgca agttgaatgt cttgctgaat gtgtctaggg 2520
gttcatcttc agtaatcgac attccactag tgccatagtt aacttcatga catgtagaca 2580
ttcaaaactt gagccttgga tgttcctgtg gacctgacag ttaaaaatat aaagaaccta 2640
ggattcaatt ccaactttct ctgtttgcct tgggttgaat aacttatctt ttggagaata 2700
gctttaagtg gcttagacac tgataaaatt cagctgtgtt gttgacgctc atctcttttg 2760
tcttacgctt agccatattt aaatcttgaa tttaatagag tctagtgaaa aaaatgagtg 2820
ggaagaatga atataaaagt aataatataa ggaaaaaggg aaagtaaact atttagaatg 2880
tagttttgtt atattcccag catttcaata tttattagtt acttgtaaat tactgtggct 2940
gtgtagttta taaatgtctg tgcactatat taattagaag accatagaac atgccagcag 3000
gttggctaat gctatggggg tttttaccac agttgccatt gtggaagaaa ttatttggta 3060
cattaataaa aaaagttggt aaaacatggt tttatacctc agtgtataag atgtgcaaga 3120
caaatatgct tatttccttt tctagaatat aagtgatatt atttgcttat gacactaaca 3180
ctattaatga caggagtcaa tcagccttta cagctatcaa aatataatga gatcccaatg 3240
atgattcttt tttactttga atgttaatta gtttgggact ttgattggct ggcaaacatt 3300
ttatcattgt cagaatttaa tttagatttc aaaaatagct tacaggattt taaacatggt 3360
gtggtattct aaagcctttt ttttaaaaaa agagatcttt ttgagagaaa caaatgagga 3420
ttgtaaagtt tggggactta cctctgtagc attgtgaaaa taaactttga ttaagctgat 3480
ttgaaaggaa aaaaaaaa 3498

<210> 12

<211>  53

<212>  DNA

<213>  Artificial

<400>  12
tcccacatca tcgatcatat tcaagagata tgatcgatga tgtgggattt ttt          53

<210>  13

<211>  61

<212>  DNA

<213>  Artificial

<400>  13
aattaaaaaa tcccacatca tcgatcatat ctcttgaata tgatcgatga tgtgggaggc    60

c                                                                    61

<210>  14

<211>  47

<212>  DNA

<213>  Artificial

<400>  14
tcccacatca tcgatcatat tcaagagata tgatcgatga tgtggga                  47

<210>  15

<211>  27

<212>  DNA

<213>  Artificial

<400>  15
gaattcagat gcagtacgcc acggggc                                        27

<210>  16

<211>  26

<212>  DNA

<213>  Artificial

<400>  16

ggtaccgcca gcatggaaaa ctgtct                                          26

<210>  17

<211>  29

<212>  DNA

<213>  Artificial

<400>  17
gaattccgat gaggctcttg ctgcttctg                                       29

<210>  18

<211>  23

<212>  DNA

<213>  Artificial

<400>  18
cgatgaggct cttgctgctt ctg                                             23

<210>  19

<211>  20

<212>  DNA

<213>  Artificial

<400>  19
gccagcatgg aaaactgtct                                                 20

<210>  20

<211>  23

<212>  DNA

<213>  Artificial

<400>  20
aactccctca agattgtcag caa                                             23

<210>  21

<211>  19

<212>  DNA

<213>  Artificial

<400>  21
gtggtcatga gcccttcca                                                    19

<210>  22

<211>  24

<212>  DNA

<213>  Artificial

<400>  22
ggtctaagct ggaatctgga catc                                              24

<210>  23

<211>  24

<212>  DNA

<213>  Artificial

<400>  23
tgcacattga gtttcatttc attg                                              24

## Description

La présente invention est relative à une sélénoprotéine impliquée dans la régulation du calcium intracellulaire.

Le calcium (Ca²⁺) est un messager intracellulaire universel qui régule des activités cellulaires très diverses telles que la fertilisation, la prolifération, la différenciation ou la mort cellulaire à travers un réseau de signalisation hautement élaboré, à partir de sources extracellulaires et intracellulaires de Ca²⁺. Les stocks intracellulaires de Ca²⁺ proviennent principalement du réticulum endoplasmique ou son homologue musculaire, le réticulum sarcoplasmique. La libération de ce Ca²⁺ dans le cytoplasme est contrôlée par le Ca²⁺ lui-même et un grand nombre de messagers tels que l'inositol-1,4,5-triphosphate (IP₃), l'acide nicotinique adénine dinucléotide phosphate (NAADP) ou le sphingosine-1-phosphate qui stimulent ou modifient la libération à travers une superfamille de canaux calciques incluant les récepteurs des IP₃ (IP₃R) et de la ryanodine (RYR) présents sur les compartiments internes (Berridge, Bootman et al. 2003). Une propriété fonctionnelle majeure de ces canaux est leur extrême sensibilité à la réduction et à l'oxydation qui peuvent stimuler ou inhiber leur activité (Feng, Liu et al. 2000). Cette sensibilité est due à l'existence de groupements sulfhydryles portés par des résidus cystéine de ces récepteurs qui peuvent être chimiquement modifiés et régulés suite à des changements de l'état redox dans la cellule.

Le Ca²⁺ peut être impliqué dans des processus physiopathologiques responsables de maladies cardiovasculaires, neurodégénératives, musculaires, métaboliques, etc., ou de tumorigenèse dans différents tissus tels que la prostate ou le pancréas. Des antagonistes des canaux calciques sont utilisés dans le traitement de certaines maladies cardiovasculaires ainsi que des pathologies neuronales ou musculaires. Plusieurs essais thérapeutiques de ces antagonistes sont actuellement en cours. La plupart de ces antagonistes ont pour cible les canaux calciques présents sur la membrane plasmique qui permettent l'entrée du Ca²⁺ dans la cellule, et notamment les canaux de type L qui peuvent être bloqués par la nimodipine. Ces approches thérapeutiques négligent l'importance des autres types de canaux (P, Q, N, T) qui jouent un rôle essentiel dans la signalisation calcique, ainsi que des canaux intracellulaires, tels que les récepteurs des IP₃ (IP₃R) et de la ryanodine (RYR), qui permettent la libération des stocks intracellulaires de Ca²⁺ dans le cytosol afin de participer à différents mécanismes de transduction.

Le PACAP (*Pituitary adenylate cyclase-activating polypeptide)* est un peptide de 38 acides aminés qui exerce des effets pléiotropiques, tant sur le système nerveux central que sur les tissus périphériques. Il est reconnu par deux types de récepteurs couplés à des protéines G : le PAC1-R, qui reconnaît sélectivement le PACAP, et deux récepteurs intestinaux vasoactifs, VPAC1-R et VPAC2-R, qui reconnaissent le PACAP et le polypeptide vasoactif intestinal avec la même affinité (Vaudry, Gonzalez et al. 2000). Parmi les modèles cellulaires sur lesquels les effets du neuropeptide PACAP ont été le plus étudiés, se trouvent les cellules sympathosurrénales dérivées de la crête neurale, qui incluent les cellules chromaffines surrénaliennes et extra-surrénaliennes, et les neurones sympathiques (Anderson 1993; Schober, Krieglstein et al. 2000). Il a été montré que le PACAP est impliqué à différents niveaux dans le développement et le contrôle de l'activité de ces cellules. Ainsi, le PACAP régule la biosynthèse et la sécrétion de catécholamines et de peptides dans les neurones sympathiques du ganglion cervical supérieur (May and Braas 1995) et dans la médullosurrénale (Isobe, Yukimasa et al. 1996; Anouar, Lee et al. 1999; Lamouche, Martineau et al. 1999; Turquier, Yon et al. 2001; Hamelink, Lee et al. 2002). De plus, le PACAP est capable d'induire la différenciation des neuroblastes sympathiques (DiCicco-Bloom, Deutsch et al. 2000), ainsi que des cellules PC12 (Deutsch and Sun 1992). La lignée PC12 (ATCC CRL-1721) est une lignée de cellules de rat dérivée d'un cancer de la médullosurrénale, le phéochromocytome, qui est capable, en réponse au NGF *(nerve growth factor),* de se différencier *in vitro* vers un phénotype neuronal (Greene and Tischler 1976; Tischler and Greene 1978). Les cellules PC12 constituent un modèle largement utilisé pour étudier la différenciation neuronale et la croissance neuritique. Outre le NGF, ces cellules peuvent répondre à d'autres facteurs trophiques, parmi lesquels le PACAP qui arrête la prolifération de ces cellules et induit un phénotype neuroendocrine et neuronal caractérisé par l'expression de marqueurs spécifiques (Grumolato, Louiset et al. 2003). Il a été montré que la différenciation des cellules PC12 par le PACAP est accompagnée par une augmentation de la concentration de calcium intracellulaire ([Ca²⁺]ᵢ) (Osipenko, Barrie et al. 2000).

Grâce à une étude utilisant des puces à ADN (Grumolato, Elkahloun et al. 2003), les Inventeurs ont maintenant identifié une sélénoprotéine impliquée dans cette augmentation de la [Ca²⁺]ᵢ. La séquence nucléotidique codant pour cette sélénoprotéine est représentée dans la liste de séquences en annexe sous le numéro SEQ ID N0: 1, et la séquence polypeptidique déduite est représentée sous le numéro SEQ ID NO: 2.

La séquence identifiée par les Inventeurs chez le rat présente une forte homologie avec une EST humaine (NM_016275 et OMIM n°607912) initialement identifiée comme codant pour une sélénoprotéine lors d'un criblage de banques de données à l'aide d'un logiciel bioinformatique qui permet l'identification d'un domaine particulier appelé SECIS (*selenocysteine insertion sequence*) (Kryukov, Kryukov et al. 1999). L'élément SECIS est présent dans la région 3' non traduite (3' UTR) des sélénoprotéines, et est responsable de la reconnaissance du triplet UGA dans la région codante de l'ARNm, comme signal d'incorporation d'un acide aminé particulier, la sélénocystéine (Sec).

Cet acide aminé, présent uniquement dans une vingtaine de sélénoprotéines identifiées à ce jour (Kryukov, Castellano et al. 2003), est une cystéine modifiée par la présence de sélénium, élément très nucléophile, à la place du soufre du groupement thiol. La Sec joue un rôle capital dans l'activité des sélénoprotéines dont la fonction a pu être déterminée. Ainsi, l'enzyme iodothyronine déiodinase, impliquée dans la maturation de l'hormone thyroïdienne, ou les glutathion peroxydases, enzymes capables d'éliminer les peroxydes dans la cellule, sont des sélénoprotéines qui agissent par un mécanisme redox très puissant grâce à un domaine catalytique renfermant la Sec (Hatfield and Gladyshev 2002).

La sélénoprotéine codée par l'EST NM_016275, initialement décrite comme comprenant 163 acides aminés, a été dénommée sélénoprotéine T (SelT) ; récemment, une séquence plus complète de SelT, comprenant 195 acides aminés, a été publiée. Cependant, la fonction biologique de cette protéine, comme celle de plus de la moitié des sélénoprotéines identifiées à ce jour, demeure inconnue (Kryukov, Castellano et al. 2003). Les seules informations disponibles concernant la SelT chez l'homme indiquent qu'elle est exprimée dans un grand nombre de tissus et d'organes ; une étude par hybridation *in situ* de trois isoformes d'un orthologue de la SelT chez le poisson zèbre pendant l'embryogenèse, indique que cette sélénoprotéine est exprimée tôt au cours du développement et qu'elle présente une distribution très étendue (Thisse, Degrave et al. 2003).

Les Inventeurs ont maintenant montré que le PACAP stimule l'expression de la SelT dans les cellules PC12 par un mécanisme très dépendant du calcium intracellulaire et extracellulaire. Ils ont en outre montré que la SelT est localisée dans l'appareil de Golgi et dans le réticulum endoplasmique, et qu'elle est responsable de l'augmentation de la concentration de calcium intracellulaire ([Ca²⁺]ᵢ) induite par le PACAP dans les cellules PC12 en cours de différenciation. Il est à noter que la forme tronquée de la SelT, décrite antérieurement par Kryukov et al. (1999, précité), ne possède pas cette activité biologique.

L'identification, pour la première fois, d'une fonction biologique de la SelT par les inventeurs permet de proposer son utilisation pour la régulation du calcium intracellulaire, et notamment dans un cadre thérapeutique.

La présente invention a donc pour objet une molécule biologique choisie parmi :
a) une sélénoprotéine, dénommée sélénoprotéine T ou SelT;
b) un polynucléotide comprenant une séquence codant pour une sélénoprotéine T, ainsi qu'une séquence SECIS située en 3' de ladite séquence codante ;

pour l'utilisation comme médicament.

Par "sélénoprotéine T", on entend ici toute sélénoprotéine possédant les caractéristiques suivantes :
(i) la séquence polypeptidique de son précurseur présente au moins 90%, de préférence au moins 95% d'identité avec l'une quelconque des séquences SEQ ID No: 2 (précurseur de la SelT de rat), SEQ ID No: 4 (précurseur de la SelT de souris), ou SEQ ID No: 6 (précurseur de la SelT humaine) ;
(ii) elle est impliquée dans la régulation de la concentration de calcium intracellulaire.

En outre, une sélénoprotéine T selon l'invention comprend de préférence un domaine transmembranaire entre les acides aminés 87 et 102, un site de N-myristoylation, deux sites de phosphorylation par la protéine kinase C et un site de phosphorylation par la caséine kinase II.

Les pourcentages d'identité de séquence auxquels il est fait référence ci-dessus sont calculés, sur une fenêtre de comparaison comprenant les 195 acides aminés de la séquence SEQ ID No: 2, 4, ou 6, choisie comme référence, en utilisant le logiciel BLASTP (Altschul, Madden et al. 1997), avec les paramètres par défaut, et sans filtrage des séquences de faible complexité.

On considèrera ici qu'une sélénoprotéine est "impliquée dans la régulation de la concentration de calcium intracellulaire" si la surexpression de cette protéine dans une cellule appropriée entraîne une augmentation de la [Ca²⁺]ᵢ. A titre d'exemple, une augmentation de 50% du niveau d'expression de ladite sélénoprotéine se traduira généralement par une augmentation d'au moins 20%, de préférence au moins 50% de la [Ca²⁺]ᵢ.

Dans le présent texte, une "séquence SECIS" est une séquence qui, lorsqu'elle est présente dans la région 3' non traduite d'un ARN messager, permet l'insertion d'une sélénocystéine lors de la lecture du codon UGA, lequel n'est alors pas lu comme un codon stop. Kryukov *et al* ont décrit un algorithme permettant l'identification de séquences SECIS (Kryukov, Castellano et al. 2003). Dans un polynucléotide selon l'invention, la séquence SECIS se trouvera donc en 3' de la séquence codante, mais en 5' des signaux d'arrêt de la transcription.

Dans une réalisation préférée de l'invention, la sélénoprotéine T utilisée comme médicament, ou codée par le polynucléotide utilisé comme médicament, correspond à la sélénoprotéine T de rat, de souris, ou humaine (séquences SEQ ID No: 2, 4 ou 6). Un médicament comportant, à titre de principe actif, une SelT de séquence SEQ ID No: 2, 4 ou 6, ou un polynucléotide codant pour une telle protéine, notamment un polynucléotide comportant une séquence choisie parmi les séquences SEQ ID No: 1, 3 et 5, constitue donc un mode de réalisation particulièrement préféré de l'invention.

De même, la séquence SECIS d'un polynucléotide utilisé comme médicament dans le cadre de la présente invention est préférentiellement choisie parmi les séquences naturellement présentes dans les gènes *SelT* de rat ou de souris (SECIS identique : SEQ ID No:7), ou humain (SEQ ID No: 8). De manière encore préférée, un tel polynucléotide comprend la séquence exacte d'un des ADNc décrits ci-dessous (SEQ ID No: 9, 10, ou 11).

Dans un médicament selon l'invention, comportant, à titre de principe actif, un polynucléotide codant pour une sélénoprotéine T, ce polynucléotide sera avantageusement compris dans un vecteur d'expression. Un tel vecteur peut être, par exemple, un plasmide comprenant un promoteur dirigeant la transcription de la séquence codante. Il peut s'agir également de tout type de vecteur viral (adénoviral, rétroviral, AAV, etc.) connu de l'homme du métier.

La présente invention porte également sur l'utilisation d'une sélénoprotéine T telle que décrite ci-dessus, ou d'un polynucléotide codant une sélénoprotéine T, ou d'un vecteur comportant un tel polynucléotide, pour la préparation d'un médicament destiné au traitement d'une maladie liée à un défaut de fonction de la SelT, se traduisant notamment par un défaut de régulation du calcium intracellulaire. L'homme du métier saura, dans une approche de thérapie génique, choisir un vecteur approprié et déterminera, en fonction de la pathologie, les organes et types cellulaires qu'il convient de cibler.

A titre de pathologies liées à un défaut de l'homéostasie du calcium intracellulaire et donc, potentiellement, à un défaut d'expression et/ou de fonction de la SelT, on peut citer les maladies suivantes :
**Maladies cardiovasculaires :**
   Cardiomyopathies
   Tachycardies
   Hypertension
   Angine de poitrine
   Insuffisance cardiaque
**Maladies neurologiques et neurodégénératives :**
   Dépression
   Epilepsie
   Maladie d'Alzheimer
   Maladie de Parkinson
   Maladie de Huntington
   Ischémie cérébrale
**Maladies musculaires et neuromusculaires :**
Différentes myopathies :
   - Dystrophies musculaires progressives (dont Duchenne de Boulogne) ;
   - Dystrophies musculaires congénitales ;
   - Myopathies congénitales ;
   - Maladies musculaires myotoniques ;
   - Maladies métaboliques du muscle (myopathies métaboliques)
**Maladies métaboliques et endocrinopathies :**
   Diabète
   Obésité
**Anomalies de l'hormonosynthèse, notamment thyroïdienne, surrénalienne, et hypophysaire.**
**Cancer :**
   Cancer de la prostate
   Cancer colorectal
   Cancer du sein
   Cancer du pancréas
   Adénomes hypophysaires
   Phéochromocytomes
   Paragangliomes
   Neuroblastomes
   Astrocytomes
   Glioblastomes
   Leucémies
**Troubles de la gamétogenèse de la reproduction :**
   Infertilité
      Troubles gonadiques

Un autre aspect de l'invention est un polynucléotide capable d'inhiber l'expression d'une sélénoprotéine telle que décrite ci-dessus, lorsqu'il est introduit ou produit dans une cellule. Il peut s'agir, par exemple, d'un ARN interférent, notamment d'un *siRNA* (*small interfering RNA*) ou d'un *shRNA* (*small hairpin RNA*), d'un ARN antisens, ou encore d'un vecteur ADN dont la transcription, dans la cellule, produira un ARN tel que mentionné ci-dessus. Un tel polynucléotide comportera de préférence une séquence d'au moins 15 nucléotides antisens d'une séquence choisie parmi les séquences des transcrits du gène *SelT* (SEQ ID No: 9, 10, et 11). Bien entendu, l'homme du métier choisira la séquence par rapport à laquelle il déterminera la séquence antisens en fonction de l'utilisation envisagée.

Un mode de réalisation particulier d'un polynucléotide capable d'inhiber l'expression d'une sélénoprotéine T est une molécule de 45 à 70 nucléotides, comportant deux régions complémentaires l'une de l'autre, d'au moins 15 nucléotides chacune, permettant le repliement de ladite molécule en épingle à cheveux. Il a en effet été démontré qu'il est possible de générer des petites molécules d'ARN interférent (*siRNA*) à partir d'une molécule d'ARN en épingle à cheveux (Yu, DeRuiter et al. 2002). Un polynucléotide particulier selon ce mode de réalisation de l'invention comprend la séquence 5'-TCCCACATCATCGATCATAttcaagagaTATGATCGATGATGTGGGA-3' (SEQ ID No: 14).

La présente invention concerne également un vecteur permettant l'introduction et/ou la synthèse, dans une cellule, d'un polynucléotide capable d'inhiber l'expression de la sélénoprotéine T. Il peut notamment s'agir d'un vecteur d'expression d'ARNi, de type plasmidique ou viral (Abbas-Terki, Blanco-Bose et al. 2002; Yu, DeRuiter et al. 2002). Un exemple de tel vecteur est le vecteur pSil-SelT décrit dans la partie expérimentale ci-dessous.

Etant donné qu'une surexpression de la SelT entraîne une augmentation du calcium intracellulaire, l'inhibition de l'expression de la SelT est également envisagée ici pour le traitement de pathologies s'accompagnant d'une élévation de la concentration intracellulaire de Ca²⁺.

Ces pathologies incluent, sans toutefois y être limitées, les maladies mentionnées plus haut, et notamment certaines maladies cardiovasculaires, certaines maladies de l'unité motrice ou neuromusculaire (par exemple, des myopathies), des maladies neurologiques (notamment, l'épilepsie ou des maladies neurodégénératives comme la maladie d'Alzheimer, la maladie de Parkinson ou l'ischémie cérébrale), certains cancers (en particulier, le cancer colorectal et le cancer de la prostate), certaines maladies métaboliques (telles que le diabète), certains défauts de la reproduction ou de la fonction immune, et des maladies liées à un vieillissement prématuré de certains tissus. Un aspect particulièrement important de l'invention est donc l'utilisation d'acides nucléiques capables d'inhiber l'expression de la sélénoprotéine T, ou de vecteurs comportant de tels polynucléotides, pour la préparation d'un médicament destiné au traitement d'une maladie liée à un défaut de régulation du calcium intracellulaire.

Toute cellule, comprenant un vecteur d'expression de la sélénoprotéine T, ou, au contraire, un polynucléotide tel que décrit ci-dessus, en particulier un vecteur, capable d'en inhiber l'expression, fait également partie de la présente invention. Le vecteur peut être soit épisomique, soit intégré dans le génome cellulaire.

L'invention porte également sur un animal non humain transgénique, de préférence un vertébré et, de manière encore préférée, un mammifère, comportant des cellules modifiées telles que décrites ci-dessus, c'est-à-dire dans lesquelles la SelT est soit surexprimée, soit, au contraire, dans lesquelles l'expression de la SelT est inhibée. Des techniques pour réaliser ce genre de modèle expérimental sont notamment décrites par Hannon et Conklin (Hannon and Conklin 2004).

Les résultats expérimentaux présentés ci-après montrent que la SelT a un rôle important dans la régulation de la concentration calcique intracellulaire. Elle pourrait être impliquée dans l'activité redox qui s'exerce sur les canaux calciques intracellulaires et qui régule leur fonction. Cette hypothèse a été confortée par le fait que la surexpression de la SelT entraîne une élévation de la [Ca²⁺]ᵢ et que l'inhibition de l'expression de cette protéine abolit la stimulation de la [Ca²⁺]ᵢ induite par un facteur trophique, le PACAP. De plus, les résultats exposés montrent que cette protéine possède une localisation subcellulaire périnucléaire fort probablement correspondant au réticulum endoplasmique/ appareil de Golgi, qui contiennent les stocks intracellulaires de Ca²⁺. La SelT pourrait être ancrée aux membranes de ces organites par l'intermédiaire du site de N-myristoylation et du domaine transmembranaire prédits par l'analyse bioinformatique. L'ensemble de ces données montre que les inventeurs ont identifié une protéine pouvant contrôler la mobilisation du calcium intracellulaire par un mécanisme original qui expliquerait la sensibilité des canaux calciques intracellulaires aux variations redox.

La caractérisation de la SelT ouvre donc de nouvelles voies de recherche sur le fonctionnement de ces canaux. En particulier, l'étude approfondie de ses caractéristiques fonctionnelles, et la détermination des différents facteurs endogènes (facteurs trophiques, neuropeptides, sélénium, etc.) ou pharmacologiques (inhibiteurs calciques, réactifs sulfhydriques, etc.) qui peuvent réguler son activité, permettront de mieux comprendre les mécanismes gouvernant la libération du calcium dans les cellules. La sélénoprotéine T, les acides nucléiques et vecteurs codant pour une sélénoprotéine T, ou capables d'en inhiber l'expression, ainsi que les cellules et animaux transgéniques comportant de tels vecteurs, tels que décrits ci-dessus, peuvent donc être utilisés conformément à la présente invention, comme outils d'analyse de la fonction des canaux calciques.

Par ailleurs, il a été montré que le sélénium, et par conséquent les sélénoprotéines qui le contiennent, joue un rôle important dans la prévention du cancer, la fonction immune, le vieillissement, la reproduction ainsi que d'autres processus physiologiques et physiopathologiques *(Ursini et al., 1999, Science 285 : 1393-1396 ; Rayman, 2000, Lancet 356 : 233- 241).* L'implication cruciale de la SelT dans la régulation de la signalisation calcique, et notamment celle du taux induit du Ca²⁺ intracellulaire mais pas du taux basal, indique que cette protéine représente une cible de choix pour développer de nouveaux agents thérapeutiques qui peuvent être utilisés dans le traitement de nombreuses pathologies s'accompagnant d'une anomalie, notamment d'une élévation de la concentration du Ca²⁺. La selénoprotéine T elle-même, les acides nucléiques dérivés du gène de la SelT, ainsi que les modèles cellulaires et animaux dans lesquels la SelT est surexprimée ou au contraire inhibée, peuvent donc être utilisés pour cribler des molécules susceptibles d'interagir, directement ou non, avec la SelT, et d'avoir un impact sur la régulation du calcium intracellulaire.

La présente invention porte donc également sur l'utilisation d'une sélénoprotéine T, d'un polynucléotide codant pour une telle protéine ou susceptible d'en inhiber l'expression, d'un vecteur comportant un tel polynucléotide, ou d'une cellule ou d'un animal comportant un tel vecteur, pour cribler des molécules susceptibles d'agir sur la concentration calcique intracellulaire.

En particulier, la sélénoprotéine T peut être utilisée dans des expériences de double hybride, pour identifier des molécules interagissant avec elle.

De même, l'homme du métier peut utiliser l'ADNc de la sélénoprotéine T, ou un fragment de cet ADNc, pour réaliser une sonde utilisable dans une puce à ADN pour étudier l'impact de composés sur la régulation de l'expression de la SelT. Une méthode de criblage, comportant une étape de mise en contact des ARN d'une cellule exposée à un composé à tester, avec une puce comportant une sonde constituée de l'ADNc de *SelT,* ou d'un de ses fragments comportant au moins 13 ou 14 nucléotides, fait donc également partie de la présente invention.

La SelT peut également être surexprimée ou inhibée dans une cellule, afin de déterminer les gènes cibles surexprimés ou inhibés, par exemple en utilisant des puces à ADN.

Un autre aspect de l'invention est une méthode de criblage de composés susceptibles d'interagir avec la sélénoprotéine T, comprenant, pour chaque composé à cribler, les étapes suivantes :
- le composé est mis en contact avec des cellules dans lesquelles la sélénoprotéine T est exprimée, et avec des cellules de même origine, dans lesquelles l'expression de la sélénoprotéine T est inhibée;
- le taux de calcium intracellulaire est mesuré dans les deux types de cellules, avant et après leur mise en contact avec le composé.

En particulier, cette méthode peut être mise en oeuvre en utilisant des cellules immortalisées. Les cellules dans lesquelles l'expression de la sélénoprotéine T est inhibée sont, par exemple, des cellules transfectées par un vecteur permettant la transcription d'un ARN interférent. Les cellules dans lesquelles la sélénoprotéine T est exprimée peuvent être des cellules contrôles, identiques à celles dans lesquelles l'expression de la SelT a été inhibée, la seule différence étant qu'elles ont été transfectées par un vecteur vide, ne permettant pas la transcription d'un ARN interférent. Alternativement, ces cellules peuvent présenter une surexpression de la SelT, par exemple en étant transfectées avec un vecteur d'expression codant pour cette protéine. Le taux de calcium intracellulaire peut être mesuré, par exemple, par la technique décrite dans la partie expérimentale ci-dessous, par mesure du ratio 405/480 nm. Une cinétique, allant de quelques heures suivant la mise en contact des cellules avec le composé à tester, à 48 heures plus tard, est de préférence réalisée. Un composé induisant une variation de la [Ca²⁺]ᵢ dans les cellules exprimant ou surexprimant la SelT, mais pas dans les cellules dans lesquelles l'expression de SelT est inhibée, est donc capable de moduler l'activité de la SelT, et constitue de ce fait un candidat potentiel pour traiter les désordres liées à une mauvaise régulation de la [Ca²⁺]_{i.}.

Un autre aspect de l'invention est une méthode pour détecter si une anomalie de l'homéostasie calcique chez un sujet est associée à une expression anormale de sélénoprotéine T, comprenant une étape de détermination de la quantité de sélénoprotéine T dans un échantillon biologique provenant du sujet présentant ladite anomalie. Cette détermination peut être effectuée soit par dosage direct de la protéine, par exemple en utilisant des anticorps marqués, soit par dosage des ARNm du gène *SelT,* par RT-PCR en temps réel.

Une autre méthode selon l'invention vise à détecter si une anomalie de l'homéostasie calcique chez un sujet est associée à une mutation du gène de la sélénoprotéine T; elle comprend une étape de recherche d'une mutation dans le gène de la sélénoprotéine T, dans un échantillon d'acide nucléique provenant du sujet présentant ladite anomalie. Cette recherche peut être effectuée soit par séquençage complet du gène et comparaison avec la séquence SEQ ID No: 11, soit, lorsque de telles mutations auront été identifiées, par des techniques plus spécifiques, telles que l'AFLP *(Amplified Fragments Length Polmymorphisms)* ou la RFLP *(Restriction Fragments Length Polmymorphisms)* connues de l'homme du métier. Les mutations peuvent être détectées dans la séquence codante, la séquence SECIS, ou les séquences de régulation du gène (promoteur, ...)

Les deux méthodes ci-dessus peuvent être utiles notamment en diagnostic, et aider le médecin à prescrire un médicament approprié.

Les exemples expérimentaux qui suivent, illustré par les figures, exposent plus en détail certains aspects de la présente invention, sans toutefois en limiter l'objet.

### Légendes des Figures :

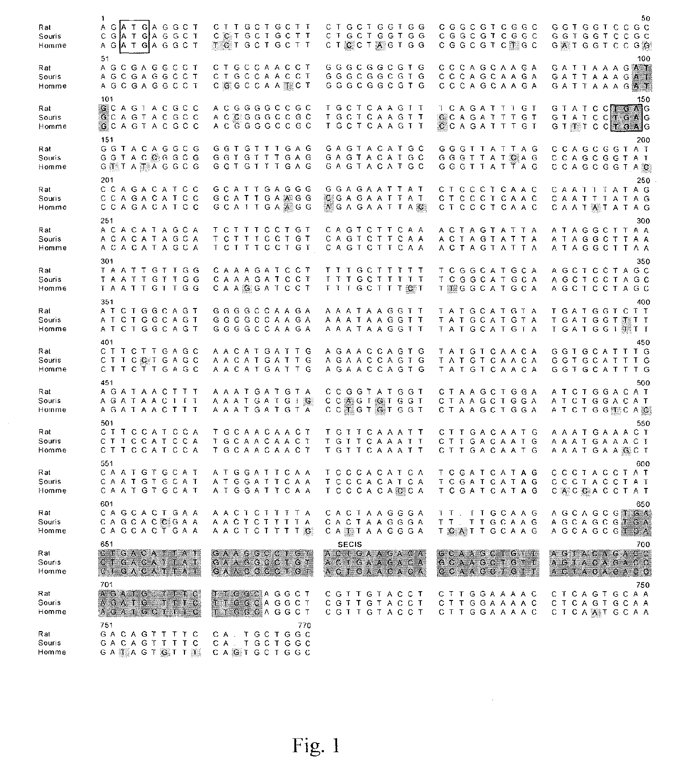

### Figure 1. Alignement des séquences nucléotidiques de la sélénoprotéine T de rat, de souris et d'homme

Les séquences de la SelT de souris (AK013022, cluster Unigene Mm.270950) et humaine (NM_016275, cluster Unigene Hs. 8148) ont été alignées avec la séquence de rat clonée par les inventeurs, à l'aide du logiciel AlignX (Vector NTI suite 5.5). La SelT de rat présente un cadre de lecture ouvert qui commence par un codon ATG (encadré, nucléotide 3 sur la figure) situé en amont d'un autre ATG (en gris, nucléotide 99) qui a été décrit comme codon initiateur de la traduction de la protéine humaine déduite d'une EST. Le premier codon ATG se trouve dans le même cadre de lecture que le second chez les trois espèces et donnerait lieu à une protéine plus longue de 32 acides aminés du côté N-terminal par rapport à la protéine humaine initialement décrite. L'ARNm de la prépro-SelT code une protéine de 195 acides aminés chez les trois espèces. Les nucléotides divergents sont indiqués par des carrés gris clair. Le codon TGA traduit en sélénocystéine est indiqué en gris encadré et le codon stop TAG en gras. La séquence SECIS *(selenocystein insertion sequence)* qui permet l'identification du codon TGA et l'insertion d'une sélénocystéine est indiquée en grisé.

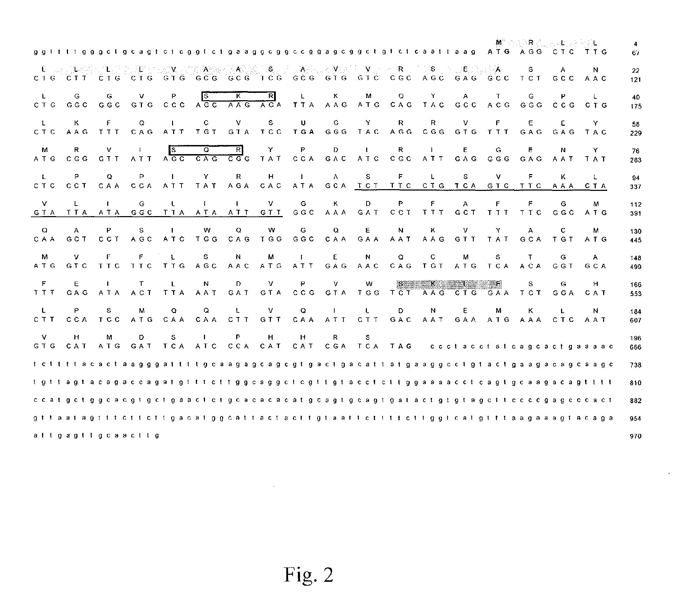

### Figure 2. Analyse de la structure de la sélénoprotéine T de rat

Le précurseur de la SelT contient un peptide signal (en gris clair, acides aminés 1 à 19) identifié grâce au logiciel SignalP V1.1 et une protéine mature de 176 acides aminés. L'analyse du profil d'hydrophobicité indique la présence d'un domaine transmembranaire entre les positions 87 et 102 (souligné). La recherche de domaines particuliers à l'aide du logiciel ScanProsite (ExPASy) a permis d'identifier deux sites potentiels de phosphorylation par la protéine kinase C (encadrés), un site potentiel de phosphorylation par la caséine kinase II (en gris) et un site potentiel de N-myristoylation (carré pointillé).

### Figure 3. Cinétique de l'effet du PACAP sur l'expression de la sélénoprotéine T

Les cellules PC12 ont été traitées pendant des temps différents par le PACAP (100 nM) et l'expression de la SelT a été mesurée par Q-RT-PCR. Les valeurs moyenness (n=3) et les erreurs standards sont représentées en pourcentage du contrôle.

### Figure 4. Effet de différents inhibiteurs de protéines kinases ou de la mobilisation de calcium sur la stimulation de l'expression de la sélénoprotéine T par le PACAP

A. Les cellules PC12 ont été incubées en l'absence (contrôle) ou en présence des différents inhibiteurs de protéines kinases pendant 1 h avant d'être traitées par le PACAP (100 nM, 2 heures). B. Les cellules PC12 ont été incubées avec les différents inhibiteurs de mobilisation calcique pendant 1 h avant d'être traitées par le PACAP (100 nM, 2 heures). Les ARN ont été extraits et les taux de SelT mesurés par Q-RT-PCR. Les résultats sont représentés en pourcentage par rapport au contrôle et constituent la moyenne de 4 échantillons (± SEM). *, P<0,05; **, P<0,01; ***, P<0,001 comparés aux contrôles.

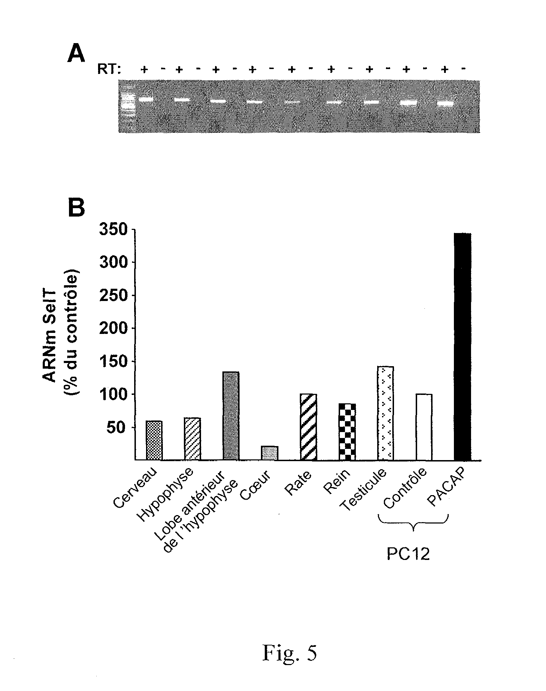

### Figure 5. Distribution de la sélénoprotéine T dans différents tissus de rat

A. Des réactions de RT-PCR (RT+) avec des amorces spécifiques de la SelT ont été effectuées sur des ARN extraits de différents tissus de rat. Les produits de PCR ont été analysés sur gel d'agarose 1,5%. Des RT-PCR contrôles ont été réalisées en absence de transcriptase inverse (RT-). B. L'histogramme représente les taux d'expression de la SelT quantifiés par Q-RT-PCR dans les différents tissus de rat, ainsi que dans les cellules PC12 traitées ou non (contrôle) par le PACAP (100 nM, 48 h). Les valeurs sont exprimées en pourcentage du taux mesuré dans les cellules PC12 non traitées. Le gène de référence utilisé est la glyceraldéhyde 3-phosphate déshydrogénase.

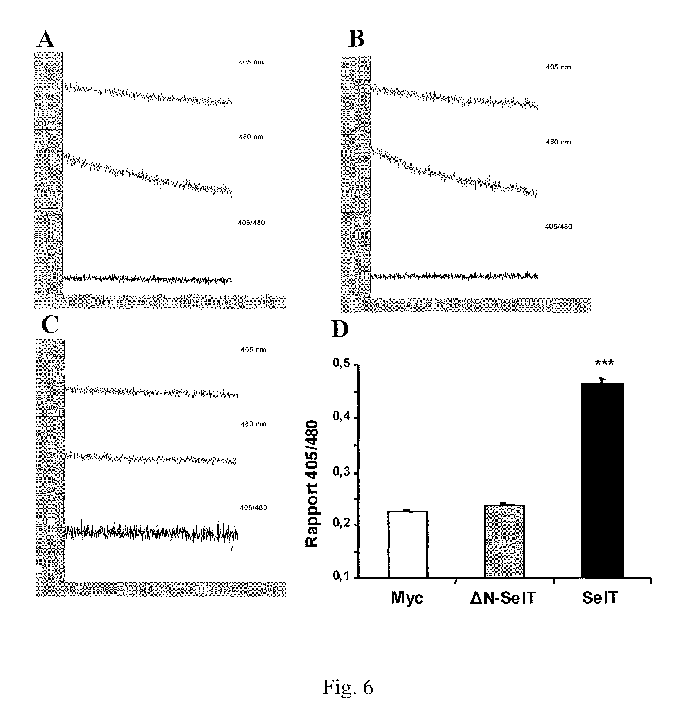

### Figure 6. Effet de la sélénoprotéine T sur la concentration du calcium intracellulaire

Les cellules PC12 ont été transfectées par le plasmide pCMV-Myc (Myc) ou le même plasmide contenant une forme tronquée (ΔN-SelT) ou la forme longue (SelT) de la SelT de rat. Deux jours après la transfection, les cellules ont été incubées 45 min à 37° C avec la sonde ratiométrique Indo-1 (2,5 µM) dans une solution saline. La concentration de calcium intracellulaire a été mesurée par microfluorimétrie. A. Le profil d'une cellule transfectée par le vecteur pCMV-Myc montrant un enregistrement en continu de la fluorescence émise à 405 nm correspondant à la sonde liée au calcium et à 480 nm correspondant à la sonde libre. Le rapport entre ces deux valeurs de fluorescence (405/480) reflète la concentration calcique intracellulaire. B, C. Les profils de deux cellules transfectées par ΔN-SelT et SelT, respectivement. D. L'histogramme représente les valeurs moyennes (± SEM) du rapport de fluorescence à 405 et à 480 nm mesuré à partir de 25 cellules pour chacune des trois conditions. Ce graphique est représentatif de deux expériences indépendantes pour un total de 62 cellules par condition.

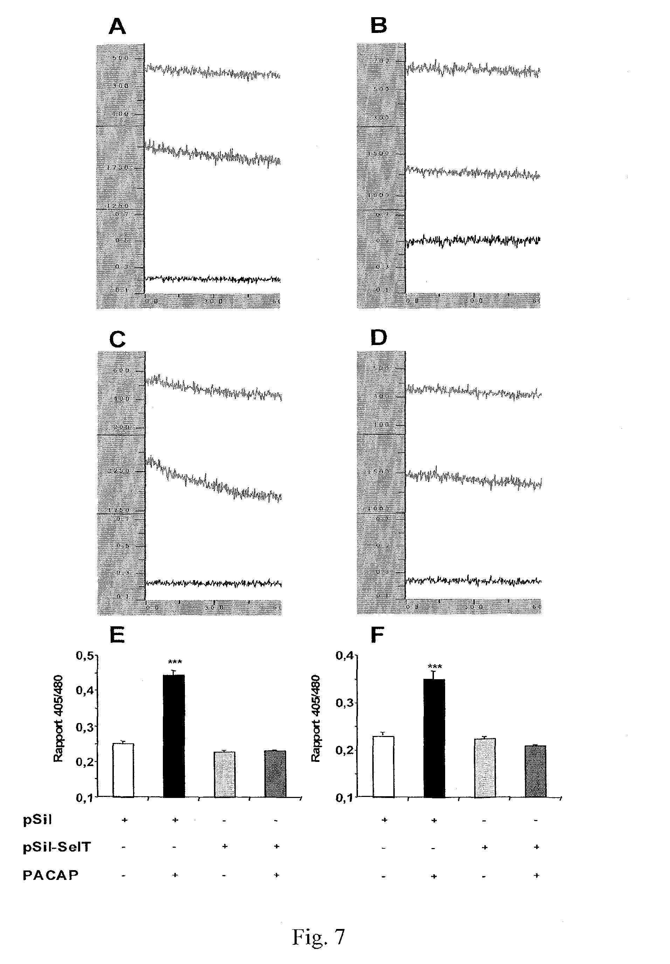

### Figure 7. Rôle de la sélénoprotéine T dans l'augmentation du calcium intracellulaire induite par le PACAP

Les cellules PC12 ont été transfectées par le vecteur d'ARNi pSilencer (pSil) ou le même vecteur contenant un insert qui permet d'inhiber l'expression de la SelT (pSil-SelT). Après transfection, les cellules ont été traitées ou non par le PACAP (100 nM) pendant 6 h ou 14 h. La concentration calcique intracellulaire a été mesurée comme décrit dans la légende de la figure 8. A, B. Profils de deux cellules transfectées par le vecteur pSil et incubées pendant 6 h en l'absence ou en présence de PACAP, respectivement. C, D. Profils de deux cellules transfectées par le vecteur pSil-SelT incubées pendant 6 h en l'absence ou en présence de PACAP, respectivement. E. L'histogramme représente les moyennes (± SEM) des rapports de fluorescence à 405 et à 480 nm obtenus à partir de cellules transfectées par les vecteurs pSil ou pSil-SelT et traitées ou non par le PACAP pendant 6 h. Ces valeurs ont été obtenues à partir de 25 cellules par condition et elles sont représentatives de trois expériences indépendantes pour un total de 109 cellules par condition. F. L'histogramme montre les résultats obtenus à partir de cellules transfectées comme décrit plus haut et traitées ou non par le PACAP pendant 14 h. Les valeurs correspondent aux moyennes (± SEM) de 25 cellules par condition et elles sont représentatives de trois expériences.

### EXEMPLES

### Matériels et méthodes

### Animaux, culture cellulaire et réactifs.

Des rats Wistar mâles, pesant entre 250 et 350 g, ont été maintenus dans des conditions contrôlées de température (22°C) et soumis à un cycle photopériodique déterminé (lumière entre 7 heures et 19 heures, obscurité le reste du temps). Les rats avaient libre accès à la nourriture du laboratoire (UAR, Epinay-sur-Orge, France) et à l'eau. Toutes les expériences ont été conduites en accord avec les recommandations du comité d'éthique français et sous le contrôle d'agents autorisés.

Les cellules PC12 de phéochromocytome de rat ont été obtenues auprès de la collection ECACC *(European Collection of Cell Culture,* Salisbury, Wiltshire, UK) et placées dans du DMEM (Sigma-Aldrich, Saint-Quentin Fallavier, France) supplémenté avec 10% de sérum de cheval (Invitrogen, Cergy Pontoise, France), 5% de sérum de veau foetal (Sigma-Aldrich), 2 mM de L-glutamine (Invitrogen), 100 unités/ml de pénicilline, et 100 µg/ml de streptomycine (Invitrogen), à 37 °C dans une atmosphère contenant 5% de CO2. Le milieu a été renouvelé tous les 2-3 jours.

Le PACAP38 a été synthétisé par la méthode en phase solide, et purifié par chromatographie liquide à haute performance, comme décrit précédemment (Chartrel, Tonon et al. 1991). L'identité du peptide a été vérifiée par spectrométrie de masse.

La chélérythrine, le L-NMMA et le BAPTA-AM proviennent de Sigma-Aldrich. Le SB203580 et la wortmannine ont été obtenus auprès de Merck Biosciences (Fontenay sous Bois, France). Le composé H89 a été fourni par Coger (Paris, France) et le composé U0126 par Promega Corp. (Charbonnières, France).

Vingt-quatre heures après leur mise en culture, la différenciation des cellules PC12 a été initiée par addition de 100 nM de PCAP38. Les inhibiteurs ont été ajoutés 1 heure après le début du traitement par le PACAP.

### Reverse transcription-PCR, clonage et séquençage.

L'ARN total a été isolé à partir des cellules PC12 indifférenciées, par la méthode Chomczynski & Sacchi (1987), en utilisant du Tri-Reagent (Sigma-Aldrich). Environ 1 µg d'ARN total a été rétro-transcrit en utilisant le système *ImProm-II™ Reverse Transcription System* (Promega), selon les instructions du fabriquant. Les amorces directe et inverse pour amplifier l'intégralité de l'ADNc codant pour la protéine SelT de rat, y compris le domaine SECIS, ont été déterminées en fonction des séquences SelT de souris (numéro d'accès AK013022) et de l'EST de rat CB556645, et ont été complétées à l'extrémité 5' par l'addition de sites de restriction EcoRI ou Kpn I (soulignés), comme décrit ci-dessous: 5'-GAATTCAGATGCAGTACGCCACGGGGC-3' (SEQ ID NO: 15) et 5'-GGTACCGCCAGCATGGAAAACTGTCT-3' (SEQ ID NO: 16).

De même, une autre amorce directe a été conçue pour amplifier une forme tronquée de la protéine SelT de rat : 5'-GAATTCCGATGAGGCTCTTGCTGCTTCTG-3' (SEQ ID NO: 17).

L'amplification, par l'ADN polymérase *Taq* (Invitrogen), a été faite en réalisant 25 cycles d'1 minute à 94 °C, 1 minute à 55 °C et 1 minute à 72 °C dans une machine Perkin-Elmer GeneAmp PCR system 9700. Les produits de PCR ont été analysés par électrophorèse sur gel d'agarose 1%, et les bandes d'ADN de la taille attendue ont été excisées à partir du gel, purifiées avec le kit d'extraction *QIAquick* (Qiagen, Courtaboeuf, France) et ligaturées dans le vecteur *pGEMT* (Promega).

Le séquençage a été réalisé avec un séquenceur d'ADN Li-Cor 4200 (ScienceTec, Les Ulis, France), en utilisant le kit Thermosequenase (Amersham Pharmacia Biotech, Orsay, France) et les amorces universelles fluorescentes T7 et M13. L'analyse des séquences a été réalisée par comparaison avec les séquences de la base de données NCBI en utilisant le logiciel BLAST (http://www.ncbi.nlm.nih.gov/BLAST/).

### PCR en temps réel.

Environ 2 µg d'ARN total de tissus de rat et de cellules PC12 ont été extraits et rétro-transcrits comme décrit ci-dessus. Un dixième du mélange réactionnel a été utilisé pour la PCR, avec les amorces ci-dessous, pour amplifier l'ADNc de la SelT: 5'-CGATGAGGCTCTTGCTGCTTCTG-3' (SEQ ID No: 18) et 5'-GCCAGCATGGAAAACTGTCT-3' (SEQ ID No: 19).

La PCR a été réalisée, comme décrit ci-dessus, et les produits ont été analyses par électrophorèse sur un gel d'agarose à 1,5%. Un contrôle de RT-PCR a été réalisé en l'absence de réverse transcriptase. Les mêmes ADNc ont été utilisés pour quantifier l'expression de la SelT, par PCR en temps réel. Les amorces spécifiques suivantes ont été conçues pour cela, en utilisant le logiciel Primer Express 2 (Applied Biosystems, Courtaboeuf, France): 5'-AACTCCCTCAAGATTGTCAGCAA-3' (SEQ ID No: 20) et 5'-GTGGTCATGAGCCCTTCCA-3' (SEQ ID No: 21) pour GAPDH; 5'-GGTCTAAGCTGGAATCTGGACATC-3' (SEQ ID No: 22) et 5'-TGCACATTGAGTTTCATTTCATTG-3' (SEQ ID No: 23) pour SelT.

La PCR en temps réel a été réalisée dans le SYBR® Green PCR Master Mix (Applied Biosystems), en présence de 300 nM des amorces spécifiques, en utilisant la machine ABI Prism 7700 (Applied Biosystems). L'efficacité relative de l'amplification des séquences de la SelT et du GAPDH a été déterminée, et les quantités d'ARNm de SelT ont été déterminées par normalisation par rapport au GAPDH, en utilisant la méthode de comparaison des C_{T} décrite dans le numéro 2 de *l'ABI Prism 7700 User Bulletin* (Applied Biosystems).

### Construction des plasmides d'expression et des vecteurs d'ARNi.

Les séquences de SelT, complète et tronquée, clonées dans pGEMT, ont été digérées avec EcoRI et SalI ou EcoRI et KpnI, respectivement. Les deux inserts ont été sous-clonés dans le vecteur d'expression pCMV-Myc (BD Biosciences, Saint-Quentin en Yvelines, France), ajoutant ainsi une étiquette Myc à l'extrémité N-terminale de chacune des formes de la SelT.

En vue d'inhiber l'expression de la SelT dans les cellules PC12, l'outil *siRNA Target* Finder, disponible sur le site internet de la société Ambion à l'adresse : http://www.ambion.com/techlib/misc/siRNA finder.html
a été utilisé pour dessiner l'oligo 1, 5'-TCCCACATCATCGATCATAttcaagagaTATGATCGATGATGTGGGAtttttt-3' (SEQ ID No: 12) et l'oligo 2, 5'-aattaaaaaaTCCCACATCATCGATCATAtctcttgaaTATGATCGATGATGTGG GAggcc-3' (SEQ ID No: 13) qui ont été hybridés et ligaturés entre les sites ApaI et EcoRI du plasmide pSilencer 1.0-U6 (Ambion, Huntingdon, UK), selon le protocole préconisé par le fabriquant, générant ainsi le vecteur pSil-SelT. Toutes les constructions ont été confirmées par séquençage.

### Transfection des cellules.

Le milieu de culture a été remplacé par un milieu dépourvu d'antibiotiques, et les cellules PC12 ont été transfectées avec 1,5-3 µg d'ADN et 2-4 µl de Lipofectamine 2000 (Invitrogen) pour les plasmides d'expression, ou 2 µg d'ADN et 4 µl de Lipofectamine 2000 pour les vecteurs d'ARNi, pendant 6 heures, selon les instructions du fabriquant. Le milieu a ensuite été changé et les cellules ont été laissées en culture pendant 2 à 5 jours.

### Immunocytochimie.

Les cellules ont été ensemencées sur des lamelles de verre (diamètre 14 mm) recouvertes de poly-L-lysine (Sigma-Aldrich) dans des plaques de 24 puits, à une densité de 10⁵ cellules/ml, et laissées en culture 48 heures avant d'être transfectées. Les cellules transfectées ont été fixées avec une solution de paraformaldéhyde à 4%, pendant 10 minutes à température ambiante, puis lavées avec du PBS. Les cellules ont ensuite été perméabilisées avec une solution contenant 1% de sérum normal de chèvre, 0.33 % de Triton X-100 et 1% de BSA, pendant 20 minutes à température ambiante. Après trois lavages au PBS, les cellules ont été incubées avec une dilution 1:100 d'un anticorps monoclonal de souris dirigé contre l'épitope Myc (BD Biosciences) dans du PBS contenant 1% de BSA, pendant 2 heures à température ambiante.

Pour déterminer si la SelT est présente dans les granules de sécrétion, les cellules ont été incubées simultanément avec un anticorps anti-Myc et un antisérum de lapin dirigé contre l'EM66 humain, à une dilution de 1:2000 (Yon, Guillemot et al. 2003). Les cellules ont été lavées trois fois avec du PBS et l'immunoréactivité a été révélée avec des γ-globulines anti-souris de chèvre conjuguées à Alexa 568 *(high cross Alexa 568-conjugated goat antimouse y -globulins,* Molecular Probes, Leiden, The Netherlands) et avec des γ-globulines anti-lapin conjuguées à Alexa 568 (Molecular Probes), à une dilution de 1:200 en PBS-1% BSA pendant 1 heure.

Pour marquer les compartiments Golgi/RE, les cellules incubées avec un anticorps anti-Myc ont été ensuite traitées avec 0.5 µM de Bodipy-TR céramide (Molecular Probes), en présence d'une dilution 1:200 dilution de γ-globulines anti-souris de chèvre conjuguées à Alexa 488 (Molecular Probes) dans du PBS-1% BSA, pendant 1 heure à température ambiante. Finalement, les cellules ont été lavées avec du PBS, rincées avec de l'eau distillée, montées avec du PBS/glycérol (1:1) et observées avec un microscope confocal à balayage laser (Leica Corp., Heidelberg, Germany) équipé avec un système optique Diaplan et un laser ionique argon/krypton. Pour vérifier la spécificité de l'immunoréaction, les anticorps primaires ou secondaires ont été remplacés par du PBS.

### Mesure du calcium.

Les cellules PC12 ont été ensemencées sur des lamelles de verre (diamètre 25 mm) recouvertes de poly-L-lysine (Sigma-Aldrich) dans des boîtes de culture de 35 mm de diamètre, à une densité de 5 X 10⁴ cellules/ml, 48 heures avant d'être transfectées. Les cellules ont ensuite été incubées à 37°C pendant 45 minutes dans l'obscurité avec 2,5 µM de sonde calcium fluorescente indo-1/acétoxyméthylester (Molecular Probes), dans une solution saline contenant 125 mM NaCl, 5,5 mM KCl, 2 mM CaCl₂, 0,8 mM MgCl₂, 10 mM HEPES, 24 mM glucose, 36,5 mM sucrose, pH 7.3 ajusté avec NaOH (Westerink and Vijverberg 2002). A la fin de l'incubation, les cellules ont été lavées deux fois avec 2 ml de solution saline à 37°C, et placées sur la plateforme d'un microscope inversé Nikon Diaphot équipé pour l'épifluorescence avec un objectif à immersion (série X 100 CF Fluor; ouverture numérique, 1.3). La concentration calcique [Ca²⁺]ᵢ a été suivie par un système de fluorimètre à double émission, comme décrit précédemment (Larcher, Lamacz et al. 1992). Succinctement, l'émission fluorescente d'indo-1/AM induite par excitation à 355 nm (lampe à xénon) a été enregistrée à deux longueurs d'onde, 405 nm correspondant à la forme complexée du Ca²⁺, et 480 nm correspondant à la forme libre, par des photomètres distincts (P1, Nikon). Le ratio 405/480 (R) a été déterminé en utilisant une carte d'acquisition de type AS1 (Notocord Systems, Croissy-sur-Seine, France). Les trois signaux (405 nm, 480 nm, et le ratio 405/480 nm) on été enregistrés en continu avec un programme JAD-FLUO (Notocord Systems).

### Résultats

### EXEMPLE 1 : CLONAGE ET CARACTERISATION DE L'ADNC DE LA SELENOPROTEINE T DE RAT

L'utilisation de *microarrays* a permis aux inventeurs de montrer que l'expression d'une *expressed sequence tag* (EST) est stimulée par le neuropeptide PACAP (*pituitary-adenylate cyclase activating polypeptide)* au cours de la différenciation des cellules PC12 de phéochromocytome de rat. Des recherches bioinformatiques ont permis d'identifier cette EST comme étant l'orthologue d'un ADNc codant une protéine humaine appelée la sélénoprotéine T (SelT), dont la fonction était alors inconnue.

Un ADNc de 767 pb, codant pour la SelT de rat, a été cloné par PCR à partir d'ARN rétro-transcrits de cellules PC12 en se basant sur les séquences d'EST recueillies dans les banques de données. L'ARNm de la SelT contient un cadre de lecture ouvert de 195 acides aminés et une séquence appelée SECIS *(selenocystein-insertion sequence*) du côté 3' non-traduit, qui permet la reconnaissance d'un codon stop UAG présent dans le cadre de lecture comme signal d'insertion d'une sélénocystéine (Sec), acide aminé dérivé de la cystéine par substitution de l'atome de soufre par un atome de sélénium dans le groupement thiol (Fig. 1). Ce 21^{ème} acide aminé est uniquement présent dans la famille des protéines appelées sélénoprotéines, qui compte une vingtaine de membres dont l'iodothyronine déiodinase, enzyme responsable de la production de l'hormone thyroïdienne ou les glutathion peroxydases, enzymes capables d'éliminer les peroxydes dans la cellule. La fonction de la majorité de ces protéines est encore inconnue. L'élément SECIS permet donc l'incorporation d'une sélénocystéine en position 49 de la protéine (Fig. 1 et 2).

La séquence nucléotidique de la SelT de rat montre une forte conservation avec les séquences de SelT de souris (98,2%) et humaine (94,7%) (Fig. 1), ce qui doit se traduire par des protéines très similaires chez ces trois espèces. La SelT de rat présente un cadre de lecture ouvert qui commence par un codon ATG (position 3) situé en amont d'un autre ATG (position 99) qui a été décrit comme codon initiateur de la traduction de la protéine humaine déduite d'une EST (Kryukov, Kryukov et al. 1999). Le premier codon ATG se trouve dans le même cadre de lecture que le second chez les trois espèces et donnerait lieu à une protéine plus longue de 32 acides aminés du côté N-terminal par rapport à la protéine humaine initialement décrite.

L'analyse de la structure de la SelT déduite de l'ADNc a révélé l'existence d'un peptide signal N-terminal de 19 acides aminés, suivi par un site potentiel de N-myristoylation. La protéine présente également des sites potentiels de phosphorylation par la protéine kinase C et la caséine kinase II. L'analyse du profil d'hydrophobicité de la SelT montre l'existence d'une région très hydrophobe entre les positions 87 et 102, pouvant représenter un domaine transmembranaire (Fig. 2).

### EXEMPLE 2 : ANALYSES CINETIQUE ET PHARMACOLOGIQUE DE L'EFFET DU PACAP SUR L'EXPRESSION DE LA SELENOPROTEINE T DANS LES CELLULES PC12.

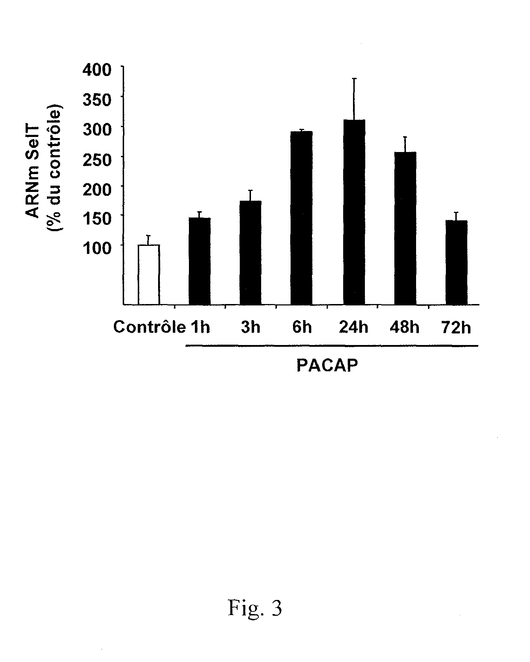

Pour mieux comprendre la régulation de l'expression de la SelT, la cinétique de la stimulation par le PACAP dans les cellules PC12 a été étudiée, ainsi que les voies de transduction impliquées dans cette régulation. Le traitement de cellules PC12 par le PACAP provoque une augmentation rapide du niveau d'ARNm de la SelT, détectable après une heure de traitement. Ceci suggère que la SelT participe aux phases précoces de la différenciation cellulaire. La stimulation par le PACAP atteint un effet maximum après 6-24 h, puis décroît jusqu'à la valeur basale après 72 h de traitement (Fig. 3) .

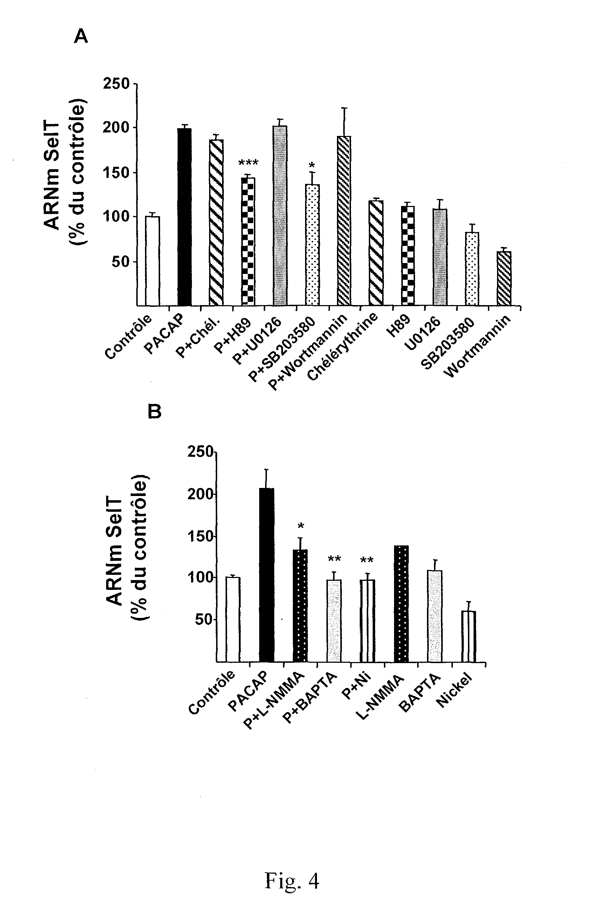

Les voies de transduction impliquées dans la régulation de l'expression de la SelT dans les cellules PC12 ont été analysées en testant l'effet de différents inhibiteurs de protéines kinases sur l'augmentation du taux d'ARNm de la SelT provoquée par le PACAP. Les résultats montrent que les inhibiteurs H89 (20 µM) et SB203580 (1 µM), qui inhibent respectivement la protéine kinase A et la p38, réduisent significativement l'effet stimulateur du PACAP sur l'ARNm de la SelT (Fig. 4A). En revanche, d'autres inhibiteurs, tels que la chélérythrine (5 µM), U0126 (10 µM) et la wortmannine (0.5 µM), qui bloquent respectivement l'activité de la protéine kinase C (PKC), de la MEK1 et de la phosphatidylinositide-3 kinase, n'ont aucun effet (Fig. 4A).

Pour déterminer l'implication des seconds messagers que sont l'oxyde nitrique et le calcium dans la régulation de l'expression de la SelT par le PACAP, les inventeurs ont analysé les effets du L-NMMA (2 mM), un inhibiteur de synthases d'oxyde nitrique, du BAPTA-AM (50 µM), un chélateur du calcium cytosolique, et du nickel (3 mM, NiCl₂), qui bloque de façon non sélective les canaux calciques. Le L-NMMA réduit significativement l'effet stimulateur du PACAP sur les niveaux d'ARNm de la SelT (Fig. 4B), ce qui montre que le PACAP est susceptible d'induire une augmentation de la production d'oxyde nitrique pour contrôler l'expression de la sélénoprotéine dans les cellules PC12. De façon remarquable, l'addition de BAPTA-AM ou de nickel a supprimé complètement l'effet stimulateur du PACAP sur l'expression de cette sélénoprotéine (Fig. 4B), ce qui montre que le calcium, tant intracellulaire qu'extracellulaire, joue un rôle crucial dans la régulation de l'expression du gène *SelT* par le PACAP dans les cellules PC12.

### EXEMPLE 3 : DISTRIBUTION TISSULAIRE DE L'ARNm DE LA SELT.

L'expression et l'abondance de l'ARNm de la SelT dans différents tissus de rat ont été étudiées par PCR et PCR en temps réel. Un transcrit unique de SelT, d'environ 700 pb, a été amplifié à partir de tous les tissus testés, *i.e*., le cerveau, l'hypophyse, le coeur, la rate, le rein, et le testicule (Fig. 5A). Les taux les plus élevés, mesurés par RT-PCR quantitative, ont été détectés dans le lobe antérieur de l'hypophyse et dans le testicule (Fig. 5B). Le niveau d'ARNm de la SelT était similaire dans tous les autres tissus étudiés, à l'exception du coeur, dans lequel le niveau d'expression était plus bas (Fig. 5B).

### EXEMPLE 4 : LA SELT EST EXPRIMEE DANS L'APPAREIL DE GOLGI ET DANS LE RETICULUM ENDOPLASMIQUE (RE).

Afin de déterminer le rôle possible de la SelT pendant la différenciation cellulaire, sa localisation subcellulaire dans les cellules PC12 a été étudiée. Pour cela, l'ADNc de cette protéine a été fusionné à l'épitope Myc et a été exprimé dans les cellules PC12 après transfection. Les inventeurs ont dans un premier temps montré par des réactions de double marquage immunocytochimique que la SelT étiquetée par Myc n'est pas localisée dans les granules de sécrétion des cellules PC12, qui sont révélés par un anticorps dirigé contre un marqueur de ces organites, le peptide EM66. En revanche, l'utilisation d'un agent pouvant marquer l'appareil de Golgi et le réticulum endoplasmique, le Bodipy-TR-ceramide (Korotkov, Kumaraswamy et al. 2001) a permis de montrer que la SelT est présente dans ces compartiments intracellulaires (appareil de Golgi et/ou RE)(Les figures correspondantes ne sont pas présentées ici).

### EXEMPLE 5 : LA SELT REGULE LA [CA²⁺]ᵢ DANS LES CELLULES PC12 EN COURS DE DIFFERENCIATION.

Les inventeurs ont recherché une éventuelle implication de la SelT dans la régulation de la concentration du Ca²⁺ dans les cellules PC12. Dans ce but, des cellules PC12 ont été transfectées avec le vecteur contenant la séquence complète de la SelT ou le plasmide exprimant la forme tronquée de la SelT décrite par Kryukov et al. (1999). La concentration de Ca²⁺ intracellulaire ([Ca²⁺]ᵢ) a ensuite été mesurée par microfluorimétrie en utilisant la sonde ratiométrique Indo-1. Il a ainsi été observé que la sur-expression de la forme entière de la SelT entraîne une élévation du niveau basal de la [Ca²⁺]ᵢ dans les cellules PC12 (Fig. 6), suggérant que cette protéine pourrait être impliquée dans la mobilisation du Ca²⁺. De façon remarquable, la forme tronquée de la SelT (Δ-SelT) n'affecte pas la [Ca²⁺] (Fig. 6, B et D) ce qui montre que la région additionnelle N-terminale identifiée par les inventeurs est nécessaire à l'activité fonctionnelle de cette sélénoprotéine.

Il a été montré que le PACAP augmente la [Ca²⁺]ᵢ dans les cellules PC12 (Osipenko, Barrie et al. 2000). Afin de déterminer si la SelT est impliquée dans l'élévation des niveaux calciques par le PACAP dans ces cellules, l'expression de la SelT a été inhibée par ARN interférence. Un vecteur permettant l'expression d'ARN court en épingle à cheveux (*short hairpin RNA,* ou *shRNA*), capable de diminuer spécifiquement l'expression de la SelT, a été utilisé. L'efficacité de ce vecteur a été démontrée par PCR en temps réel. En effet, 5 jours après transfection par ce vecteur, les cellules PC12 traitées par le PACAP ou non traitées montraient une diminution de 60% de l'expression de la SelT, par comparaison avec les cellules transfectées avec le vecteur vide. Les inventeurs ont montré qu'un traitement par le PACAP, pendant 6 ou 14 heures, entraîne une augmentation significative de la [Ca²⁺]ᵢ dans les cellules PC12, et que cet effet est complètement abrogé dans les cellules dans lesquelles l'expression de la SelT a été réprimée, dans lesquelles les niveaux de la [Ca²⁺]ᵢ sont comparables à ceux observés dans les cellules contrôles (Fig. 7). La surexpression de la SelT est donc une étape cruciale dans le mécanisme déclenché par le PACAP pour induire une augmentation de la [Ca²⁺]ᵢ pendant la différenciation des cellules PC12. L'ensemble de ces données démontre que la SelT est une nouvelle protéine de régulation du taux du Ca²⁺ intracellulaire et doit donc jouer un rôle crucial dans les multiples processus contrôlés par la signalisation calcique dans différentes cellules.

### Discussion

Les résultats ci-dessus ont permis d'identifier la protéine SelT comme un acteur crucial dans l'augmentation de la [Ca²⁺]ᵢ induite par le PACAP pendant la différenciation des cellules PC12. Cette étude constitue le premier rapport sur l'activité biologique de la SelT et démontre pour la première fois l'implication d'une sélénoprotéine dans l'homéostasie calcique.

Le clonage de l'ADNc de rat a permis d'identifier un nouveau codon initiateur potentiel, situé 96 nucléotides en amont du début de la région codante de l'ARNm décrite précédemment (Kryukov, Kryukov et al. 1999). La séquence additionnelle est très conservée dans les gènes de SelT chez l'homme et la souris, ce qui montre que la SelT correspond très probablement à une protéine comportant 32 acides aminés supplémentaires. L'extrémité N-terminale de la SelT nouvellement identifiée contient un peptide signal et des sites putatifs de N-myristoylation et phosphorylation par la PKC qui sont probablement impliqués dans l'adressage correct de cette protéine et la régulation de son expression. De plus, la présence d'un domaine très hydrophobe entre les positions 87 et 102 du précurseur suggère que la SelT pourrait être une protéine transmembranaire. Il est intéressant de noter que dans une étude ayant pour but l'identification par bio-informatique des protéines sécrétées et des protéines transmembranaires, la SelT a été classée parmi les protéines transmembranaires (Clark, Gurney et al. 2003).

Comme première approche pour déterminer l'activité biologique de la SelT, les inventeurs ont étudié son expression dans différents tissus de rat et les mécanismes de sa régulation par le PACAP dans les cellules PC12. Ils ont montré une distribution étendue des ARNm de SelT, avec des niveaux plus élevés dans le lobe antérieur de l'hypophyse et dans le testicule, ce qui suggère que cette sélénoprotéine exerce une fonction générale dans plusieurs types cellulaires. Le niveau d'ARNm de SelT dans les cellules PC12 indifférenciées est comparable aux concentrations mesurées dans les différents tissus de rat examinés. Cependant, le PACAP induit une augmentation d'un facteur 3 du niveau d'ARNm de SelT dans les cellules PC12 après 48 heures de traitement. Une étude cinétique a montré que le niveau d'ARNm de SelT atteint un maximum après 6-24 h, puis retourne au niveau basal après 72 h de traitement par le PACAP, ce qui indique que le rôle de cette sélénoprotéine pendant la différenciation des cellules PC12 est probablement transitoire.

Le PACAP régule différentes voies de transduction dans les cellules PC12. Il a été montré que la croissance de neurites dans ces cellules, induite par le PACAP, dépend de l'activation de la phospholipase C et de la MAPK, mais pas de la protéine kinase A (Lazarovici, Jiang et al. 1998). Au contraire, la protéine kinase A est nécessaire à la stimulation de l'expression du gène de la tyrosine hydroxylase induite par le PACAP dans les cellules PC12 (Nanmoku, Isobe et al. 2000). L'augmentation des niveaux de cAMP par le PACAP dans ces cellules est associée à une augmentation de l'expression du neuropeptide Y et du gène de la pro-enképhaline (Colbert, Balbi et al. 1994; Monnier and Loeffler 1998), ainsi qu'à une augmentation du calcium dans les cellules PC12 (Osipenko, Barrie et al. 2000). Cette augmentation de la [Ca²⁺]ᵢ est à son tour nécessaire aux effets immédiats et à long terme du PACAP sur la sécrétion de catécholamines par les cellules PC12 (Taupenot, Mahata et al. 1999). Pour examiner les voies de transduction impliquées dans la régulation de l'expression de la SelT par le PACAP, plusieurs inhibiteurs de protéines kinases et de messagers secondaires ont été utilisés. Cela a révélé que l'effet du PACAP sur les niveaux d'ARNm de SelT dépend de la protéine kinase A, de p38 et de l'oxyde nitrique, mais pas de la phospholipase C ni de la MEK, ce qui indique que les mécanismes activés par le neuropeptide dans les cellules PC12 pour augmenter l'expression de la SelT sont distincts de ceux responsables de la croissance des neurites. Ces expériences ont également montré que le calcium provenant du milieu extérieur ou relargué à partir de sources intracellulaires est crucial pour la régulation de la SelT. L'implication de l'AMPc et du calcium dans l'augmentation de l'expression de la SelT induite par le PACAP dans les cellules PC12 suggère que les mécanismes de régulation de cette protéine présentent des similarités avec ceux activés lors de la sécrétion de catécholamines.

La localisation sub-cellulaire de la SelT a été étudiée en utilisant une protéine étiquetée par Myc. Cela a montré que la SelT n'est pas co-localisée avec le peptide EM66 dérivé de la sécrétogranine II, marqueur pour les granules de sécrétion (Taupenot, Harper et al. 2003), ce qui suggère que la SelT ne participe pas, au moins directement, au processus de sécrétion hormonale. Au contraire, la SelT étiquetée a été détectée dans une région périnucléaire des cellules PC12, marquées avec un marqueur fluorescent, ce qui indique que la protéine SelT native est probablement localisée dans le RE/appareil de Golgi.

Les inventeurs ont également montré que la surexpression de la SelT dans les cellules PC12 induit une augmentation de la [Ca²⁺]ᵢ par rapport aux cellules contrôles, ce qui laisse penser que cette protéine pourrait médier le relarguage du calcium stocké dans des compartiments intracellulaires.

La forme tronquée de la SelT, qui ne contient pas l'extrémité N-terminale nouvellement identifiée, est sans effet sur la [Ca²⁺]ᵢ, indiquant ainsi que les 32 acides aminés N-terminaux nouvellement identifiés sont indispensables à l'activité biologique de la protéine.

L'inhibition de l'expression de la SelT par ARN interférence a aboli l'augmentation de la [Ca²⁺]ᵢ induite par le PACAP dans les cellules PC12.

Ces résultats indiquent que l'augmentation de l'expression de la SelT est une condition nécessaire et suffisante pour l'augmentation de la [Ca²⁺]ᵢ qui accompagne la différenciation des cellules PC12 induite par le PACAP.

Il a été décrit que l'état redox de sulfhydryles critiques situés du côté cytoplasmique du récepteur ryanodine de type 1 pouvait altérer tant son activité de canal que sa réponse à des modulateurs (Hamilton and Reid 2000; Oba, Murayama et al. 2002). On peut donc imaginer que le motif redox putatif contenu dans la SelT (Kryukov, Kryukov et al. 1999; Hatfield and Gladyshev 2002) module l'activité de canaux calciques intracellulaires, notamment IP₃R et RYR, par un mécanisme redox.

Les résultats présentés ici révèlent que la SelT, qui est elle-même régulée par le Ca²⁺ dans les cellules PC12, joue un rôle crucial dans ce processus, permettant ainsi d'identifier une cible d'intérêt pour le contrôle des mécanismes de prolifération / différenciation / fonction d'un grand nombre de types cellulaires. En effet, le PACAP est un facteur trophique ubiquiste agissant à différents niveaux de l'organisme au cours du développent et chez le sujet adulte. De plus, les inventeurs ont montré que l'expression de la SelT est également ubiquiste, ce qui suggère fortement qu'il s'agit là d'une cible universelle dont la fonction peut être contrôlée par des agents thérapeutiques dans des pathologies associées à des dysfonctionnements des voies de signalisation intracellulaires impliquant le Ca²⁺.

Compte tenu du rôle central que joue le Ca²⁺ intracellulaire dans différentes voies de signalisation physiologiques et physiopathologiques, la découverte d'une protéine capable d'augmenter la concentration de la [Ca²⁺]ᵢ au cours de la différenciation et dont l'inhibition abolit l'effet d'un facteur trophique sur la [Ca²⁺]ᵢ peut avoir des applications concrètes dans plusieurs domaines. Ainsi, la protéine identifiée peut représenter une cible thérapeutique de choix pour développer des médicaments pouvant agir sur différentes voies de signalisation impliquant le calcium dans des pathologies diverses telles que les maladies cardiovasculaires, neurologiques ou musculaires. Le gène identifié peut être utilisé en thérapie génique pour corriger des défauts de signalisation calcique. Enfin, cette protéine peut être utilisée en recherche en tant qu'outil d'analyse de la fonction des canaux calciques.

### REFERENCES

Abbas-Terki, T., W. Blanco-Bose, et al. (2002). "Lentiviral-mediated RNA interference." Hum Gene Ther **13**(18): 2197-201.

Altschul, S. F., T. L. Madden, et al. (1997). "Gapped BLAST and PSI-BLAST: a new generation of protein database search programs." Nucleic Acids Res **25**(17): 3389-402.

Anderson, D. J. (1993). "Molecular control of cell fate in the neural crest: the sympathoadrenal lineage." Annu Rev Neurosci **16**: 129-58.

Anouar, Y., H. W. Lee, et al. (1999). "Both inducible and constitutive activator protein-1-like transcription factors are used for transcriptional activation of the galanin gene by different first and second messenger pathways." Mol Pharmacol **56**(1): 162-9.

Berridge, M. J., M. D. Bootman, et al. (2003). "Calcium signalling: dynamics, homeostasis and remodelling." Nat Rev Mol Cell Biol **4**(7): 517-29.

Chartrel, N., M. C. Tonon, et al. (1991). "Primary structure of frog pituitary adenylate cyclase-activating polypeptide (PACAP) and effects of ovine PACAP on frog pituitary." Endocrinology **129**(6): 3367-71.

Clark, H. F., A. L. Gurney, et al. (2003). "The secreted protein discovery initiative (SPDI), a large-scale effort to identify novel human secreted and transmembrane proteins: a bioinformatics assessment." Genome Res **13**(10): 2265-70.

Colbert, R. A., D. Balbi, et al. (1994). "Vasoactive intestinal peptide stimulates neuropeptide Y gene expression and causes neurite extension in PC12 cells through independent mechanisms." J Neurosci **14**(11 Pt 2): 7141-7.

Deutsch, P. J. and Y. Sun (1992). "The 38-amino acid form of pituitary adenylate cyclase-activating polypeptide stimulates dual signaling cascades in PC12 cells and promotes neurite outgrowth." J Biol Chem **267**(8): 5108-13.

DiCicco-Bloom, E., P. J. Deutsch, et al. (2000). "Autocrine expression and ontogenetic functions of the PACAP ligand/receptor system during sympathetic development." Dev Biol **219**(2): 197-213.

Feng, W., G. Liu, et al. (2000). "Transmembrane redox sensor of ryanodine receptor complex." J Biol Chem **275**(46): 35902-7.

Greene, L. A. and A. S. Tischler (1976). "Establishment of a noradrenergic clonal line of rat adrenal pheochromocytoma cells which respond to nerve growth factor." Proc Natl Acad Sci U S A **73**(7): 2424-8.

Grumolato, L., A. G. Elkahloun, et al. (2003). "Microarray and suppression subtractive hybridization analyses of gene expression in pheochromocytoma cells reveal pleiotropic effects of pituitary adenylate cyclase-activating polypeptide on cell proliferation, survival, and adhesion." Endocrinology **144**(6): 2368-79.

Grumolato, L., E. Louiset, et al. (2003). "PACAP and NGF regulate common and distinct traits of the sympathoadrenal lineage: effects on electrical properties, gene markers and transcription factors in differentiating PC12 cells." Eur J Neurosci **17**(1): 71-82.

Hamelink, C., H. W. Lee, et al. (2002). "Coincident elevation of cAMP and calcium influx by PACAP-27 synergistically regulates vasoactive intestinal polypeptide gene transcription through a novel PKA-independent signaling pathway." J Neurosci **22**(13): 5310-20.

Hamilton, S. L. and M. B. Reid (2000). "RyR1 modulation by oxidation and calmodulin." Antioxid Redox Signal **2**(1): 41-5.

Hannon, G. J. and D. S. Conklin (2004). "RNA interference by short hairpin RNAs expressed in vertebrate cells." Methods Mol Biol **257**: 255-66.

Hatfield, D. L. and V. N. Gladyshev (2002). "How selenium has altered our understanding of the genetic code." Mol Cell Biol **22**(11): 3565-76.

Isobe, K., N. Yukimasa, et al. (1996). "Pituitary adenylate cyclase-activating polypeptide induces gene expression of the catecholamine synthesizing enzymes, tyrosine hydroxylase and dopamine beta hydroxylase, through 3',5'-cyclic adenosine monophosphate- and protein kinase C-dependent mechanisms in cultured porcine adrenal medullary chromaffin cells." Neuropeptides **30**(2): 167-75.

Korotkov, K. V., E. Kumaraswamy, et al. (2001). "Association between the 15-kDa selenoprotein and UDP-glucose:glycoprotein glucosyltransferase in the endoplasmic reticulum of mammalian cells." J Biol Chem **276**(18): 15330-6.

Kryukov, G. V., S. Castellano, et al. (2003). "Characterization of mammalian selenoproteomes." Science **300**(5624): 1439-43.

Kryukov, G. V., V. M. Kryukov, et al. (1999). "New mammalian selenocysteine-containing proteins identified with an algorithm that searches for selenocysteine insertion sequence elements." J Biol Chem **274**(48): 33888-97.

Lamouche, S., D. Martineau, et al. (1999). "Modulation of adrenal catecholamine release by PACAP in vivo." Am J Physiol **276**(1 Pt 2): R162-70.

Larcher, A., M. Lamacz, et al. (1992). "Effect of vasotocin on cytosolic free calcium concentrations in frog adrenocortical cells in primary culture." Endocrinology **131**(3): 1087-93.

Lazarovici, P., H. Jiang, et al. (1998). "The 38-amino-acid form of pituitary adenylate cyclase-activating polypeptide induces neurite outgrowth in PC12 cells that is dependent on protein kinase C and extracellular signal-regulated kinase but not on protein kinase A, nerve growth factor receptor tyrosine kinase, p21(ras) G protein, and pp60(c-src) cytoplasmic tyrosine kinase." Mol Pharmacol **54**(3): 547-58.

May, V. and K. M. Braas (1995). "Pituitary adenylate cyclase-activating polypeptide (PACAP) regulation of sympathetic neuron neuropeptide Y and catecholamine expression." J Neurochem **65**(3): 978-87.

Monnier, D. and J. P. Loeffler (1998). "Pituitary adenylate cyclase-activating polypeptide stimulates proenkephalin gene transcription through AP1- and CREB-dependent mechanisms." DNA Cell Biol **17**(2): 151-9.

Nanmoku, T., K. Isobe, et al. (2000). "Orexins suppress catecholamine synthesis and secretion in cultured PC12 cells." Biochem Biophys Res Commun **274**(2): 310-5.

Oba, T., T. Murayama, et al. (2002). "Redox states of type 1 ryanodine receptor alter Ca(2+) release channel response to modulators." Am J Physiol Cell Physiol **282**(4): C684-92.

Osipenko, O. N., A. P. Barrie, et al. (2000). "Pituitary adenylyl cyclase-activating peptide activates multiple intracellular signaling pathways to regulate ion channels in PC12 cells." J Biol Chem **275**(22): 16626-31.

Schober, A., K. Krieglstein, et al. (2000). "Molecular cues for the development of adrenal chromaffin cells and their preganglionic innervation." Eur J Clin Invest **30 Suppl 3:** 87-90.

Taupenot, L., K. L. Harper, et al. (2003). "The chromogranin-secretogranin family." N Engl J Med **348**(12): 1134-49.

Taupenot, L., M. Mahata, et al. (1999). "Time-dependent effects of the neuropeptide PACAP on catecholamine secretion : stimulation and desensitization." Hypertension **34**(5): 1152-62.

Thisse, C., A. Degrave, et al. (2003). "Spatial and temporal expression patterns of selenoprotein genes during embryogenesis in zebrafish." Gene Expr Patterns **3**(4): 525-32.

Tischler, A. S. and L. A. Greene (1978). "Morphologic and cytochemical properties of a clonal line of rat adrenal pheochromocytoma cells which respond to nerve growth factor." Lab Invest **39**(2): 77-89.

Turquier, V., L. Yon, et al. (2001). "Pituitary adenylate cyclase-activating polypeptide stimulates secretoneurin release and secretogranin II gene transcription in bovine adrenochromaffin cells through multiple signaling pathways and increased binding of pre-existing activator protein-1-like transcription factors." Mol Pharmacol **60**(1): 42-52.

Vaudry, D., B. J. Gonzalez, et al. (2000). "Pituitary adenylate cyclase-activating polypeptide and its receptors: from structure to functions." Pharmacol Rev **52**(2): 269-324.

Westerink, R. H. and H. P. Vijverberg (2002). "Ca(2+) -independent vesicular catecholamine release in PC12 cells by nanomolar concentrations of Pb(2+)." J Neurochem **80**(5): 861-73.

Yon, L., J. Guillemot, et al. (2003). "Identification of the secretogranin II-derived peptide EM66 in pheochromocytomas as a potential marker for discriminating benign versus malignant tumors." J Clin Endocrinol Metab **88**(6): 2579-85.

Yu, J. Y., S. L. DeRuiter, et al. (2002). "RNA interference by expression of short-interfering RNAs and hairpin RNAs in mammalian cells." Proc Natl Acad Sci U S A **99**(9): 6047-52.

## Revendications

1. Médicament comprenant, à titre de principe actif, une molécule biologique choisie parmi :
a) une sélénoprotéine, dénommée SelT, dont la séquence peptidique présente au moins 90%, de préférence au moins 95% d'identité avec l'une quelconque des séquences de SelT de rat (SEQ ID No: 2), de souris (SEQ ID No: 4), ou humaine (SEQ ID No: 6) ;
b) un polynucléotide comprenant une séquence codant pour une sélénoprotéine telle que définie ci-dessus, ainsi qu'une séquence SECIS située en 3' de ladite séquence codante.

2. Médicament selon la revendication 1, **caractérisé en ce que** la séquence de ladite SelT est choisie parmi les séquences SEQ ID No: 2, 4 et 6.

3. Médicament selon la revendication 1 ou la revendication 2, **caractérisé en ce qu'**il comprend un polynucléotide dans lequel la séquence codante de ladite SelT est choisie parmi les séquences SEQ ID No: 1, 3 et 5.

4. Médicament selon l'une quelconque des revendications 1 à 3, **caractérisé en ce qu'**il comprend un polynucléotide dans lequel la séquence SECIS est choisie parmi les séquences SEQ ID No: 7 et 8.

5. Médicament selon l'une quelconque des revendications 1 à 4, **caractérisé en ce qu'**il comprend un polynucléotide comprenant une séquence choisie parmi les séquences SEQ ID No: 9, 10, et 11.

6. Polynucléotide isolé, **caractérisé en ce qu'**il est capable d'inhiber l'expression de la sélénoprotéine T.

7. Polynucléotide selon la revendication 6, **caractérisé en ce qu'**il comporte une séquence d'au moins 15 nucléotides antisens d'une séquence choisie parmi les séquences SEQ ID No: 9, 10, et 11.

8. Polynucléotide selon la revendication 7, **caractérisé en ce qu'**il s'agit d'une molécule de 45 à 70 nucléotides, comportant deux régions complémentaires l'une de l'autre, d'au moins 15 nucléotides chacune, permettant le repliement de ladite molécule en épingle à cheveux.

9. Polynucléotide selon la revendication 8, **caractérisé en ce qu'**il comprend la séquence SEQ ID No: 14.

10. Vecteur permettant l'introduction et/ou la synthèse, dans une cellule, d'un polynucléotide selon l'une quelconque des revendications 6 à 9.

11. Médicament comprenant, à titre de principe actif, un polynucléotide selon l'une quelconque des revendications 6 à 9, ou un vecteur selon la revendication 10.

12. Cellule comprenant un vecteur selon la revendication 10.

13. Animal non humain transgénique comportant des cellules selon la revendication 12.

14. Utilisation d'une sélénoprotéine T, d'un polynucléotide codant pour une sélénoprotéine T, d'un vecteur d'expression de la sélénoprotéine T, d'un polynucléotide selon l'une quelconque des revendications 7 à 10, d'un vecteur selon la revendication 11, d'une cellule selon la revendication 13, ou d'un animal selon la revendication 14, comme outil d'analyse de la fonction des canaux calciques.

15. Utilisation d'une sélénoprotéine T, d'un polynucléotide codant pour une sélénoprotéine T, d'un vecteur d'expression de la sélénoprotéine T, d'un polynucléotide selon l'une quelconque des revendications 7 à 10, d'un vecteur selon la revendication 11, d'une cellule selon la revendication 13, ou d'un animal selon la revendication 14, pour cribler des molécules susceptibles d'agir sur la concentration calcique intracellulaire.

16. Méthode de criblage de composés susceptibles d'interagir avec la sélénoprotéine T, comprenant, pour chaque composé à cribler, les étapes suivantes :
- le composé est mis en contact avec des cellules dans lesquelles la sélénoprotéine T est exprimée, et avec des cellules de même origine, dans lesquelles l'expression de la sélénoprotéine T est inhibée;
- le taux de calcium intracellulaire est mesuré dans les deux types de cellules, avant et après leur mise en contact avec le composé.

17. Méthode pour détecter si une anomalie de la concentration calcique intracellulaire chez un sujet est associée à une expression anormale de sélénoprotéine T, comprenant une étape de détermination de la quantité de sélénoprotéine T dans un échantillon biologique provenant du sujet présentant ladite anomalie.

18. Méthode pour détecter si une anomalie de la concentration calcique intracellulaire chez un sujet est associée à une mutation du gène de la sélénoprotéine T, comprenant une étape de détection d'une mutation dans le gène de la sélénoprotéine T, dans un échantillon d'acide nucléique provenant du sujet présentant ladite anomalie.
